# EUROPEAN PATENT APPLICATION

(11) **EP 4 722 284 A1**
(43) Date of publication of application: **08.04.2026**
(21) Application number: 24203787.7
(22) Date of filing: 01.10.2024
(51) Int. Cl.: C08K 5/3435, C07D 211/00, C08J 3/22, C08K 5/00, C08L 77/06, C08L 77/02, C08L 23/04, C08L 23/12

(54) **LOW MELTING CARBOXAMIDE HALS FOR IMPROVED STABILIZATION OF POLYMERS OR RESINS**

(71) Applicant: CLARIANT INTERNATIONAL LTD, 4132 Muttenz (CH)
(72) Inventor: SCHEIBELEIN, Christoph, 86152 Augsburg (DE); STEFFANUT, Pascal, 68730 Michelbach le bas (FR); AMNUAYPORNSRI, Sureerut, 10400 Bangkok (TH); SCHALLER, Thomas, 86391 Stadtbergen (DE)
(74) Representative: Zusammenschluss Clariant

(57) **Abstract**

The invention relates to a low molecular weight HALS stabilizer (LMW-HALS) for enhancing the processing and long-term stability and the heat resistance, light resistance and chemical resistance of polymer or resin._The present invention also relates to a stabilizer masterbatch and a composition comprising such stabilizer and a polymer or resin.

## Description

### Technical field

The present invention relates to a low molecular weight HALS stabilizer (LMW-HALS stabilizer) for enhancing the processing and long-term stability, such as the heat, light and oxidation resistance of thermoplastic and duroplastic polymers such as polyamides, polyolefins or polyurethanes.

The present invention also relates to a LMW-HALS stabilizer masterbatch and a polymer compositions comprising such LMW-HALS stabilizer.

Further the present invention relates to thermoplastic and duroplastic coating systems comprising the inventive LMW-HALS stabilizers for enhancing the processing stability and the heat, light and chemical resistance of such coating systems.

### Background of the invention

Thermoplastic polymers, such as polyamides and polyolefins are widely used in industry to produce fibers or molded bodies by spinning or injection molding. Polyolefins, especially polypropylene and polyethylene are also used in hotmelt adhesives.

Thermoplastic and duroplastic polymers such as polyolefins, polyurethanes, polyurethane resins, acryl resins, polyester resins, epoxide resins or alkyd resins are used for coating systems, such as powder coatings or physically drying coating systems (water or solvent-based) and/or reactive coating systems (e.g. 2K systems, thermally cross-linking systems, UV systems).

The service-life of such polymers is dependent on (1) raw materials used in the manufacturing process and the manufacturing process itself, (2) the melt processing and the additives used for stabilizing the polymer (stabilizers) against damage brought about by melt-processing and (3) environmental weathering factors.

Melt-processing is applied in extrusion processes, such as spinning processes and injection molding procedures or for hot melt adhesive preparation and processing.

Synthetic polyamides are typically melt-processed at temperatures between 240°C and 300°C, and especially PA 6 is processed at only 240°C, which is roughly 20°C above its melting point. The typical melt-processing temperature for polyethylene (PE) is in the range of 180 - 240 °C and for polypropylene (PP) it lies in the range of 190 - 270°C. Heat stability issues can arise in the temperature range between 150°C and 300°C in that polyamides or polyolefins are decomposed by heat and oxygen, which cause the decomposition of the polymeric chains and therefore impact color changes and the loss of favorable mechanical properties. Therefore, heat stabilization against thermo-oxidative degradation processes is required.

Hot melt adhesives for industrial scale applications further require long-term heat stabilization during the melt-processing. In such applications, heat stress appears during the production of the hotmelt adhesive and furthermore during the application of hotmelt adhesives. During production, hotmelt adhesives components are homogenized in a batch or continuous extrusion mixing process. The industrial application to the target substrates requires to store the hot melt adhesive in a molten state over several days in a storage tank. Polypropylene based hot melt adhesives (e.g. Licocene^{®} PP 2602) need temperatures of up to 180°C. Under these conditions long-term heat stabilization is mandatory to avoid unfavoured degradation such as chain scission or cross-linking.

Environmental weathering factors arise during the use of molded and extruded goods at ambient temperatures under heat, light and oxygen exposure. These negative long-term influences are not as intense as those caused by melt-processing, but over a longer period of time, they cause significant ageing and discoloration of the material and loss of the mechanical properties. Under these conditions, polymers such as polyolefins and polyamides require long-term stabilization against exposure to heat, light and oxygen.

To reduce these unfavored degradation effects, the processing temperatures should be kept as low as possible to reduce thermo-oxidative degradation processes, and stabilizing additives (stabilizers) such as antioxidants and hindered amine light stabilizers (HALS) need to be added to the polymer composition. Antioxidants are of great importance during the melt-processing stage, whereas long-term stability at ambient temperature is increasingly being supplied by hindered amine light stabilizers (HALS).

Antioxidants are often called primary or secondary antioxidants, depending on their mechanism of action.

Primary antioxidants based on phenols (such as Hostanox O 3, Irganox 1010 and Irganox 1076) and secondary antioxidants like phosphites (such as Irgafos 168) or phosphonites (such as Hostanox P-EPQ) are used for process stabilization by scavenging radicals and peroxides formed at high temperatures. These antioxidants have the necessary kinetics to prevent oxidation processes rapidly at high temperatures. It has proven advantageous to use mixtures of primary and secondary antioxidants, such as Irganox B 215 (mixture of Irganox 1010 and Irgafos 168).

However, a disadvantage of these antioxidants is that they are consumed for stabilization and therefore, the long-term processing of hot melt adhesives requires-large amounts of antioxidants for long-term heat stabilization. The relatively high concentrations of antioxidants additionally cause a negative impact on secondary properties, such as discoloration or the formation of increased amounts of "nonintentional added substances" (NIAS), e.g. the ARVIN substances. These become more and more unwanted in plastic articles. For example, in medical applications the primary antioxidants need to be replaced by hydroxylamines or HALS.

Therefore, hindered amine light stabilizers (HALS stabilizers), which act as self-regenerating radical scavengers are used in polymers to reduce their degradation during long-term heat exposure and to enhance long-term stability at ambient temperatures. Especially sterically hindered cyclic amines are widely used in industry as polymer stabilizers.

In most cases they are a derivative of 2,2,6,6-tetramethylpiperidine.

They are generally produced using 2,2,6,6-tetra-methyl-4-piperidinyl (TAA) or from its derivatives, such as 2,2,6,6-tetramethylpiperidin-4-ol (TAAol) and 2,2,6,6-tetramethylpiperidin-4-amine (TAD), N-butyl-2,2,6,6-tetramethyl-piperidin-4-amine (Bu-TAD) or N,N'-bis(2,2,6,6-tetramethyl-4-piperidyl)hexane-1,6-diamine (HMBTAD). HALS compounds scavenge efficiently free radicals formed in plastics articles and in coatings or lacquers, especially at the surface, where minor or no protection through the UV absorber is given. This process has been extensively studied and is essentially a cyclic chain breaking antioxidant process which is known as the Denisov cycle.

Further, HALS stabilizers were currently described as thermal degradation and long-term heat stabilizers, which show process stabilizing effects, but also an unfavorable steady decline in the mechanical properties of the stabilized polymer during a short period of time. (P. Gijsman, J. Polymer Degradation and Stability, 2017, Vol. 145, p 2 - 10)

WO9743335 discloses N,N'-bis(2,2,6,6-tetramethyl-4-piperidinyl)-1,3-benzenedicarboxamide, which is also known as the commercial product Nylostab^{®} S-EED (NS).

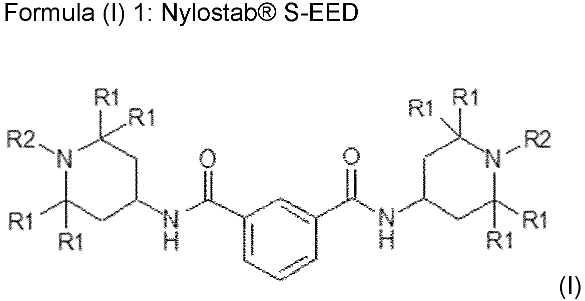

Nylostab^{®} S-EED is performing as multipurpose additive and allows process stability as well as light, heat and oxidation resistance. Due to its chemical structure, Nylostab^{®} S-EED is highly suitable for polyamides. It is usually introduced upstream during the polymerization process of polyamides due to its reasonably good solubility in ε-caprolactam.

Alternatively, it can be added in downstream processes, such as compounding, extrusion, spinning, injection molding or masterbatch preparation.

However, the high melting point of about 274°C limits its use in compounding and extrusion to high melting polymers, as processing temperatures in extrusion must be adapted to at least 280°C to ensure dispersibility in the downstream processes.

Due to the dispersibility issues as described above Nylostab^{®} S-EED is not used for other types of polymers being usually processed at lower temperatures like polyolefins and therefore limits its efficiency as light or heat stabilizer in polyolefins.

Further, increased temperatures are unfavorable due to increased energy consumption and increased oxidation processes causing discoloration and loss of mechanical properties.

EP1556350 describes a general process for the preparation of benzene-carboxamide HALS compounds based on TAD and IPC building blocks with generic combinations of R1 and R2 substituents. Specific combinations of R1 and R2 substituents beside Nylostab^{®} S-EED which show special physical and chemical features are not described.

JPH0733738 discloses further generic structures based on piperidine amine alkylated TAD derivatives and various benzene carboxylic acid building blocks. In these structures, the piperidine N of the tetramethylpiperidine (TAD) derivatives is always alkylated. This alkylation is performed using unwanted carcinogenic, mutagenic, reprotoxic (CMR) substances such as formaldehyde. Non-alkylated N-2,2,6,6-tetramethyl-4-piperidinyl 1, 3 benzene carboxamide based HALS compounds derived from isophthalic acid were neither explicitly described nor produced.

The non-N-alkylated benzene carboxamide based HALS compounds disclosed in the prior art often have high melting points which require high melt-processing temperatures and therefore enhance the thermal-oxidative stress on a polymer or resin. Further they have a poor compatibility with polyolefins and a poor solubility in solvents used for solvent-based coatings.

The piperidine-N-alkylated benzene carboxamide based HALS compounds have lower melting points but require carcinogenic, mutagenic, reprotoxic (CMR) substances for their manufacturing and where not tested for their compatibility and solubility.

Therefore, the problem addressed by the present invention is to provide a benzene carboxamide based HALS stabilizer which broadens the application field in terms of polymer or resin type, melt-processing temperature and processing level (upstream or downstream). Further, it is desirable to provide a benzene carboxamide based HALS stabilizer that is compatible with low melting polymers and soluble in coatings formulations of different polarity.

Thus, it is desirable to provide an improved multi-purpose benzene carboxamide HALS stabilizer that provides good or improved stabilizing properties against both: thermo-oxidative degradation processes during melt-processing and environmental weathering factors. Further, it should be compatible with various types of polymers and with coating formulations of different polarities.

Such benzene carboxamide based HALS stabilizer should be easy to incorporate, either by itself or by providing it as a masterbatch and should not negatively affect the properties of a polymer, polymer composition or coating formulation.

### Subject Matter of the invention

This problem has been solved by the compound of formula (II), which provides new chemical modifications to the class of N,N'-bis(2,2,6,6-tetramethyl-4-piperidinyl) - benzenedicarboxamides. 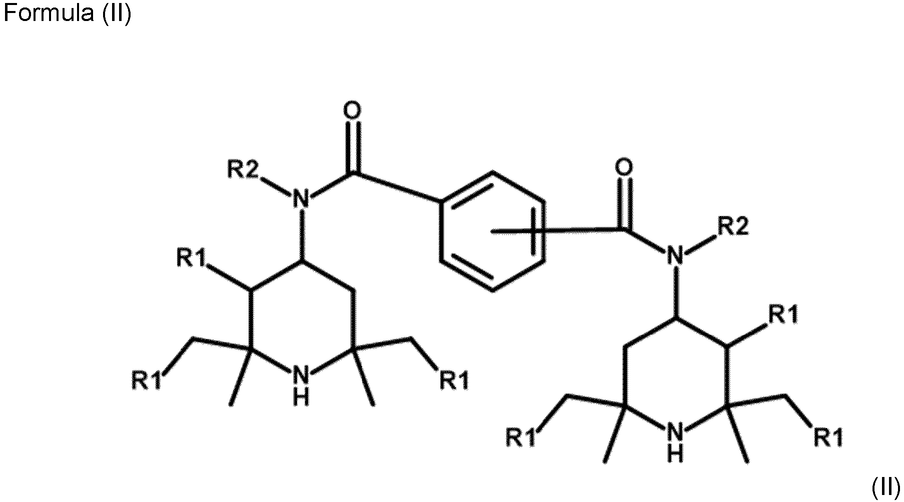 whereas
- R₁,: is selected from the group consisting of H and linear alkyl groups having from 1 to 3, more preferably from the group consisting of H and linear alkyl groups having from 1 to 2, carbon atoms; and
- R₂: is selected from linear, branched or cyclic alkyl groups having from 2 to 8 carbon atoms, more preferably from 3 to 6 carbon atoms, particularly preferably from 4 to 5 carbon atoms.

It was found that the compound of formula (II), an alkylated amid version of certain sterically hindered 4-amino piperidine compounds reacted with benzenedicarboxylic acid (BCA) derivates, wherein the piperidine amine is not alkylated, has a lower melting point than comparable structures such as Nylostab^{®} S-EED and extends their application field by enhancing the heat, light and oxidation resistance of various thermoplastic or duroplastic polymers, which are used in coating systems or processed in melt-processing and used for injection molding or hot melt adhesives.

Compounds of formula (II) were found to have a (a) process-stabilizing effect on polypropylene and polyamides (e.g. PA 6) and (b) a long term heat stabilizing effect in hot melt adhesives and (c) increased solubilities in solvents applicable for solvent based coatings, i.e. they protect the polymer during high temperature associated processing, although this property is actually attributed to classical phenol- or phosphite-based antioxidants.

Compounds of formula (II) are synthesized by condensation of a benzenedicarboxylic acid chloride or it's dimethyl ester with a sterically hindered piperidine amine of formula (III): 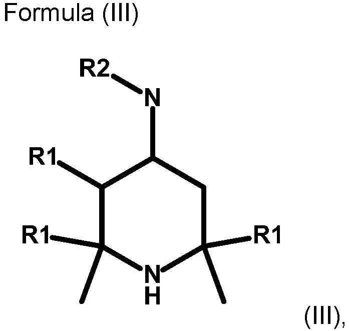 whereas
- R₁,: is selected from the group consisting of H and linear alkyl groups having from 1 to 3, more preferably from the group consisting of H and linear alkyl groups having from 1 to 2, carbon atoms; and
- R₂: is selected from linear, branched or cyclic alkyl groups having from 2 to 8 carbon atoms, more preferably from 3 to 6 carbon atoms, particularly preferably from 4 to 5 carbon atoms.

Such stabilizers can be added to all kind of thermoplastic or duroplastic polymers to improve their melt-processing stability and their long-term heat, light and chemical stability.

Accordingly, a polymer composition comprising a thermoplastic or duroplastic polymer and compound of formula (II) is also an object of the invention, as is a process for its preparation.

The compound of formula (II) can also be provided in the form of a masterbatch of a polymer, such as polyamide or a polyolefin, so that the invention also relates to a masterbatch comprising a compound of formula (II).

Accordingly, another object of the invention is a coating system comprising (a) a thermoplastic or duroplastic polymer such as polyolefins or polyurethane resins, acryl resins, polyester resins, epoxide resins or, alkyd resins and (b) a compound of formula (II).

The invention accordingly also provides a method for the stabilization of thermoplastic or duroplastic polymers, preferably of polyamides or polyolefins, which comprises admixing (a) the polymer before or during processing or (b) the monomers used for the polymerization of the thermoplastic or duroplastic polymer with an effective amount of the compound of formula (II).

### Detailed Description of the invention

Preferably the compound of formula (II) is a compound of formula (IV): 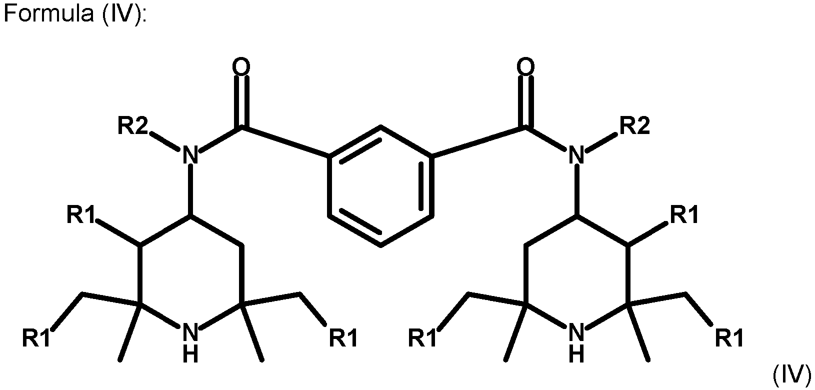 wherein the benzenedicarboxylic acid building block (BCA) is derived from isophthalic acid (IPA).

The compound of formula (IV) is particularly preferred because it has a significantly lower melting point compared to the compound, which is derived from terephthalic acid. Additionally, the compound of formula (IV) has a better solubility in monomers such as ε-caprolactam and hexamethylene diamine. Furthermore, the compound of formula (IV) has a significantly better solubility in organic solvents, such as xylene, n-butylacetate and MPA than the compound, which is derived from terephthalic acid and thus, extends its application to solvent-based coating systems.

In a preferred embodiment of the compound of formula (IV) R1 is either hydrogen or a methyl group, more preferably hydrogen.

In an alternative preferred embodiment, R2 is a butyl group, because this substance combines a particularly good solubility in various solvents (e.g. xylene, MPA, butyl acetate) with pronounced stabilizing effects on thermoplastic and duroplastic polymers and further combines pronounced stabilizing properties in the melt-processing of thermoplastic polymers, in particular of polyolefins and polyamides, with a good compatibility with thermoplastic polymers.

In a particularly preferred embodiment, the compound of formula (IV) is a compound, wherein R1 is hydrogen and R2 is butyl, preferably n-butyl. This compound combines very good solubilities in certain monomers and solvents with a lower melting point and good stabilizing properties, without impairing other desired properties of the polymer, such as viscosity, optical properties or mechanical strength.

The compound of formula (IV) wherein R1 is hydrogen and R2 is n-butyl (formula (V), Stabilizer 1, "S1") is usually present in its an amorphous phase but can also be isolated in various crystalline structures.

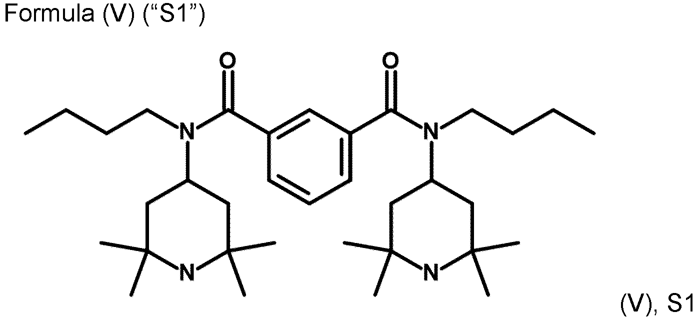

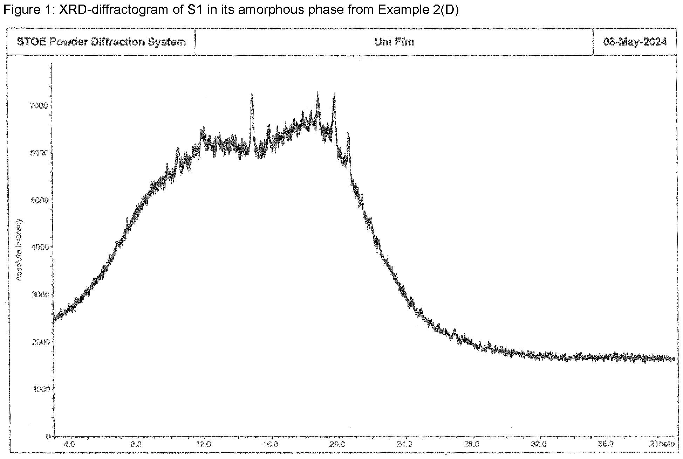

S1 in its amorphous phase is characterized by the characteristic amorphous scattering pattern in the x-ray powder diffractogram, measured with Cu-Kα1 radiation at a 2θ angle between 3° and 25° and the absence of distinct reflexes. Depending on sample preparation, low intensity, distinct reflexes of the α-, β- and γ-phase can be present (Figure 1). The glass transition temperature of the amorphous phase is between 26 - 32 °C.

The amorphous phase can be difficult to handle in an industrial scale due its glass transition temperature close to room temperature resulting in an unwanted tackiness at ambient conditions, which leads to agglomeration and bonding of particulate products such as pills, granules, powder and other product finishings.

Preferably S1 can be crystallized into at least two different pure polymorphic phases (α- and β-phase) and a third polymorphic phase (γ-phase) can be obtained as a mixture with the α-phase. The polymorphic phases can be characterized by characteristic reflections in the x-ray powder diffractogram, measured with Cu-Kα1 radiation. The reflections are classified by their intensities. The Intensity ("Int") denotes the intensities from visual estimation as follows: ss = very strong, s = strong, m = moderate (medium), w = weak, vw = very weak, br = broad or splitted peak.

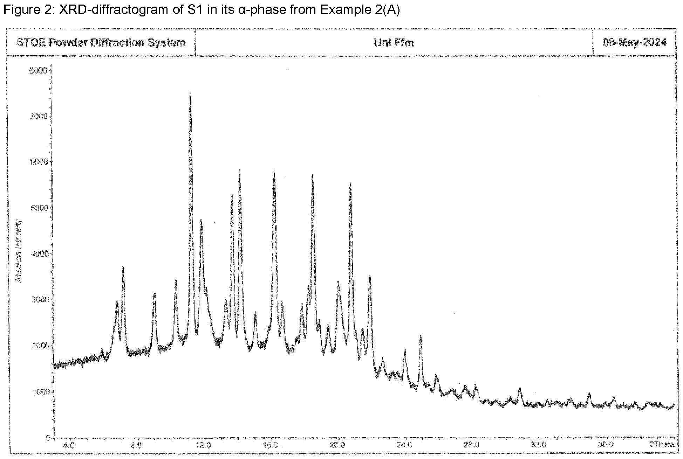

S1 in its α-phase is characterized by reflections with strong intensities in the x-ray powder diffractogram, measured with Cu-Kα1 radiation in transmission mode on a STOE STADI P diffractometer at 2θ angles of 11.3°, 11.9°, 13.7°, 14.2°, 16.2°, 18.6° and 20.8°, preferably with additional reflections with moderate intensities at 2θ angles of 6.8°, 7.2°, 9.0°, 10.3°, 13.3°, 15.1°, 16.7°, 17.9°, 20.1°, 21.9° and 24.9° degrees (Figure 2). The corresponding melting point for the α-phase is around 119 - 120 °C.

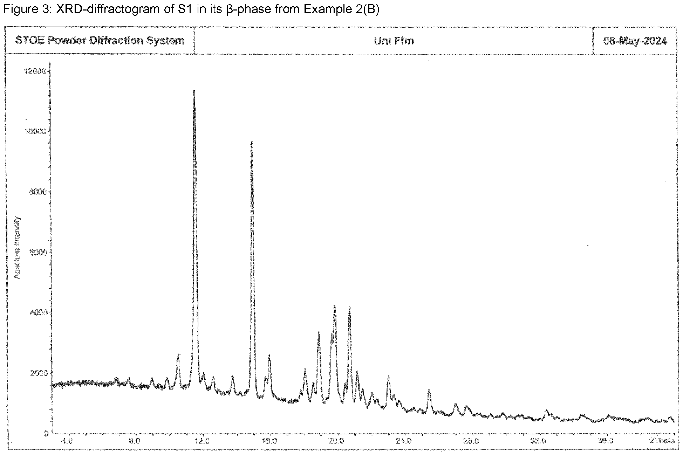

S1 in its β-phase is characterized by reflections with strong intensities in the x-ray powder diffractogram, measured with Cu-Kα1 radiation in transmission mode on a STOE STADI P diffractometer at 2θ angles of 11.6° and 15.0°, preferably with additional reflections with moderate intensities at 2θ angles of 18.9°, 19.6° and 20.7°, especially preferred with additional reflections with weak intensities at 2θ angles of 10.5°, 16.0°, 18.1°, 21.2°, 23.0° and 25.4° (Figure 3). The corresponding melting point for the β-phase is around 129 - 131 °C.

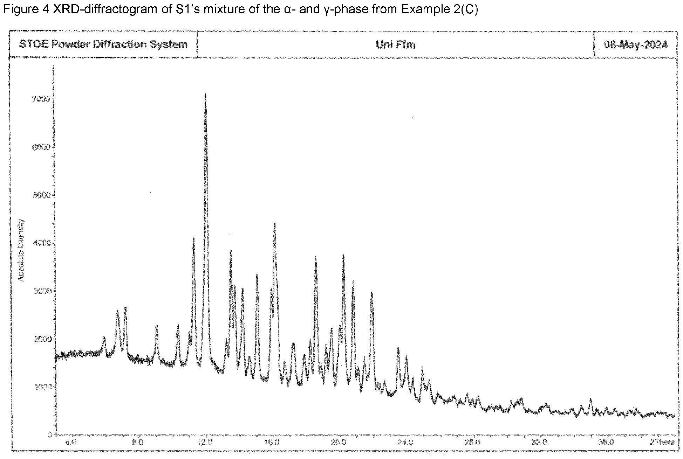

S1 in its γ-phase can be extracted from the mixture of α- and γ-phase by subtracting the signals attributed to the α-phase (Figure 4). The γ-phase is characterized by a reflection with very strong intensity in the x-ray powder diffractogram, measured with Cu-Kα1 radiation in transmission mode on a STOE STADI P diffractometer at a 2θ angle of 12.1°, and reflections with strong intensities at 2θ angles of 13.5°, 16.8° and 20.3°, and preferably with additional reflections with moderate intensities at 5.9°, 17.2° and 23.5°, and especially preferred with additional peaks of weak intensities at 2θ angles of 11.0°, 19.2° and 24.4°.

The polymorphic phases are preferred for handling the compound on an industrial scale, as they prevent agglomeration and improve flowability of particulate products, because all three phases (α- and β-phase, as well as the γ-phase or the mixed α- and γ-phase) have melting points that are many times higher than the glass transition temperature of the amorphous phase.

Compounds of formula (II) are obtainable by condensation of a benzenedicarboxylic acid chloride or it's dialkyl ester, preferably isophthalic acid chloride or the dialkyl esters, particularly preferably isophthalic acid chloride (IPC) or dimethyl isophthalate ester, most preferably isophthalic acid chloride (IPC) with two equivalents of a sterically hindered piperidine amine of general formula (III), following processes described in the published applications WO2004016591 or WO2017005413. The preparation of the isophthalic acid variant using IPC is preferred, because higher yields and better purity of the inventive compound can be obtained.

The alkylated amino tetramethyl piperidine of formula (III) is commercially available and is obtainable by a reductive amination of triacetone amine (TAA) using primary alkylamines, like n-butylamine or hexamethylene-1,6-diamine. These processes are well-known industrial methods.

In a preferred embodiment of the process, R1 of formula (III) is hydrogen.

In an alternative preferred process for the preparation of the compound of formula (II), the substituent R2 is a butyl group.

The process according to the invention can be carried out particularly efficiently, if the molar ratio of BCA to the compound of formula (III) is from 1:1,8 to 1:2,5, preferably it is from 1:1,8 to 1:2,1, more preferably it is from 1:1,9 to 1:2.

Preferably the condensation is carried out in liquid phase, whereas the solvent is either an aromatic solvent, such as toluene, chlorobenzene, xylene, mesitylene, or an aliphatic solvent, such as hexane, cyclohexane, heptane, n-octane, iso-octane, cyclooctane, decane or an alcohol selected from the homologous row of alkanols from CHsOH to C₈H₁₇OH, such as methanol, ethanol, n-propanol, iso-propanol, n-butanol, iso-butanol, tert-butanol or a mixture of that alcohol with water. The preferred aromatic solvent is xylene, the preferred aliphatic solvent is cyclohexane. If alkanol solvents are used methanol, ethanol and propanol or mixtures of these alcohols and water are preferred. Further, xylene, n-propanol, isopropanol or a mixture of xylene or n- or iso-propanol and water are more preferred; even more preferred is xylene or isopropanol or a mixture of 60 - 80 % vol of xylene or isopropanol and 20 -40 % vol of water.

The addition of an effective amount of the inventive compound to a composition comprising a thermoplastic or duroplastic polymer enhances the heat, light and oxidative resistance of the composition without impairing other desired properties, such as viscosity, mechanical strength, or optical properties. Therefore, the compound of formula (II) is used to stabilize thermoplastic or duroplastic polymers against degradation, discoloration and/or yellowing of polymers caused by heat, light or oxidation. The compositions comprising the compound of formula (II) are typically used for melt extrusion processes, hot melt formulations or coating systems.

The compound of formula (II) is advantageously added to such a composition in an amount of 0.01 to 10.0 wt%, preferably of 0.05 to 5.0 wt%, more preferably of 0.08 to 2.5 wt%, in particular of 0.1 to 1.0 wt%, based on the total weight (100 wt%) of the total composition. Thus, the composition shows a lower yellowing tendency and has better mechanical properties after melt-processing, long-term heat storage or exposure to environmental weathering factors simulated by weatherometer stability tests.

The polymer stabilized by the inventive compound of formula (II) can be any thermoplastic or duroplastic polymer or resin known in the art, such as polyolefin homopolymers and copolymers, thermoplastic elastomers, polyesters and polyester resins, polyurethanes and polyurethane resins, such as 2-K-PUs, polyalkylene terephthalates, polysulfones, polyimides, polyphenylene ethers, styrenic polymers and copolymers, polycarbonates, acrylic polymers and acrylic resins, such as 1-K- acryl resins, polyamides, polyacetals, halide-containing polymers, epoxides and epoxide resins, polyester resins and alkyd resins and biodegradable polymers. Mixtures of different polymers, such as polyphenylene ether/styrenic resin blends, polyvinyl chloride/ ABS or other impact modified polymers, such as methacrylonitrile and α-methylstyrene containing ABS, and polyester/ ABS or polycarbonate/ ABS and polyester plus some other impact modifier may also be used.

Such polymers are available commercially or may be made by a variety of polymerization processes well known in the art including solution, high pressure, slurry and gas phase using various catalysts including Ziegler-Natta, single-site, metallocene or Phillips-type catalysts.

The compound of formula (II) is particularly useful in thermoplastic polymers, preferably in polyolefins, due to the relatively low temperatures at which thermoplastic polymers are often processed and/or used.

The polyolefins, typically ethylene or propylene based polymers, have a density in the range of from 0.86 g/cc to 0.97 g/cc, preferably in the range of from 0.88 g/cc to 0.965 g/cc, more preferably in the range of from 0.895 g/cc to 0.96 g/cc, even more preferably in the range of from 0.900 g/cc to 0.95 g/cc, yet even more preferably in the range from 0.910 g/cc to 0.940 g/cc. The polyolefins typically have a molecular weight distribution, as defined as weight average molecular weight to number average molecular weight (Mw/Mn) between about 1.5 and about 15, preferably from about 2 to about 10, more preferably from 2.2 to 8, even more preferably from 2.2 to 5, and most preferably from 2.5 to 4. The ratio of Mw/Mn can be measured by gel permeation chromatography techniques well known in the art. The polymers of the present invention in one embodiment have a melt index (MI) as measured by ASTM-D-1238-E in the range from 0.01 dg/min to 1000 dg/min, preferably from about 0.01 dg/min to about 100 dg/min, more preferably from about 0.1 dg/min to about 50 dg/min, and most preferably from about 0.1 dg/min to about 10 dg/min.

The polyolefins may be produced by, for example, polymerization of olefins in the presence of Ziegler-Natta catalysts. They may also be produced utilizing chromium catalysts or single site catalysts, e.g., metallocene catalysts. The polyolefins are synthesized using for example, ethylene, propylene, 1-butene, 1-hexene and 1-octene and other 1-olefins comprising up to 20 carbon atoms, preferably ethylene and propylene or a mixture thereof.

In another embodiment, the compound of formula (II) is suitable for use in polyolefins that are used in hot melt adhesives.

The compound of formula (II) is further particularly useful in polyamides, due to the good solubility in the monomers, the low melting point and the melt-processing stabilization effects.

Polyamides stabilized according to the invention contain amide linkages (-CONH-) in the polymer backbone, which are synthesized from diamines, dicarboxylic acids, lactams, or amino acids. These polymers could be classified as aliphatic, semi-aromatic and aromatic polyamides. Examples include nylon 6 (PA 6), nylon- 6.6 (PA 66), nylon-6.10, nylon 6.11, nylon-6.12, nylon 11, nylon 12, and copolymers such as nylon-6.6/6, nylon-6.6/6.10, 6/11, 6/12; polyether-polyamide block copolymers, poly(m-phenylene-isophthalamide), poly(p-phenylene terephthalamide), and the like all of which are commercially available from a variety of sources.

The methods for synthesizing polyamide polymers, comprise step-growth polymerization, ring-opening polymerization, anionic polymerization, cationic polymerization, or interfacial polymerization. The monomers used for these polymerizations are for example ε-caprolactam, ω-aminolauric acid, laurolactam, hexamethylenediamine, 11-aminoundecanoic acid, terephthalic acid, terephthalic chloride, isophthalic acid, isophthalic chloride, adipic acid, tetramethylenediamine, phenylenediamine.

The method includes using the stabilization system with formulated polyamides, including conventional impact toughened polyamide, and reinforced polyamides containing glass fiber, mineral and glass/mineral combinations. The invention can be practiced with polyamides regardless of the polymer morphology. Amorphous, semicrystalline or highly crystalline polyamides as well as blends of different crystallinity are benefited. Commercially available polyamide resins are known from sources such as BASF, DSM, Evansville, Ind. and E. I. duPont de Nemours; formulated polyamides are available, for example, from Ferro Corp., A. Schulman and Akron; reinforced polyamides are available from DSM and Westlake.

Polymers, especially polyamides or polyolefins, stabilized with compound of formula (II) are useful in melt-processing operations as film, sheet, and fiber extrusion and co-extrusion as well as blow molding, injection molding and rotary molding. Films include blown or cast films formed by co-extrusion or by lamination useful as shrink film, cling film, stretch film, sealing films, oriented films, snack packaging, heavy duty bags, grocery sacks, baked and frozen food packaging, medical packaging, industrial liners, membranes, etc. in food-contact and non-food contact applications.

Fibers include melt spinning, solution spinning and melt blown fiber operations for use in woven or non-woven form to make textiles, fleeces, filters, diaper fabrics, medical garments, geotextiles, etc. Extruded articles include medical tubing, wire and cable coatings, geomembranes, and pond liners.

Molded articles include single and multi-layered constructions in the form of bottles, tanks, large hollow articles, rigid food containers and toys, etc.

In another embodiment, the compound of formula (II) is suitable for use in polymers based on acrylic acids, such as acrylic acid, methacrylic acid, methyl methacrylic acid and ethacrylic acid and esters thereof may also be used. Such polymers include polymethylmethacrylate, and ABS-type graft copolymers wherein all or part of the acrylonitrile type monomer has been replaced by an acrylic acid ester or an acrylic acid amide. Polymers including other acrylic-type monomers, such as acrolein, methacrolein, acrylamide and methacrylamide may also be used.

The present invention further provides a composition, comprising an aforementioned polymer or a mixture thereof, and the compound of formula (II), advantageously in an amount of 0.05 to 10.0 wt%, preferably of 0.1 to 5.0 wt%, more preferably of 0.2 to 2.5 wt%, in particular of 0.25 to 1.0 wt%, based on the overall weight (100 wt%) of the composition. Preferably the polymer is a polyamide or a polyolefin or a polyurethane or a styrenic polymer or an acrylic polymer, more preferably the polyamide is PA 6 or PA 66 and the polyolefin is a polyethylene or polypropylene homo- or copolymer.

The composition may be in the form of a powder, a pellet, a fiber or any desired shaped article, for example a self-supporting film/sheeting, a container, an injection molded article, a coating, a pipe or a profile.

The present invention further provides a masterbatch comprising from 5 to 95 wt%, preferably from 10 to 90 wt%, more preferably from 20 to 80 wt%, in particular from 30 to 70 wt% of the compound of formula (II) and from 95 to 5 wt%, preferably from 90 to 10 wt%, more preferably from 80 to 20 wt%, in particular from 70 to 30 wt% of a matrix polymer. The polymer that is used for the masterbatch is identical to or at least compatible with the polymer to be stabilized, and preferably is a polyolefin or polyamide. The stabilizer masterbatch is obtainable by mixing the individual constituents, preferably in powder or pellet form, and optionally by melting them together and subsequent pelletization.

The inventive compositions are obtainable via a process comprising the admixture of the compound of formula (II) or the aforementioned masterbatch with the above-described polymers in an appropriate processing equipment, for example in an extruder or kneader. The incorporation of the compound of formula (II) or the aforementioned masterbatch may be used either before or during melt-processing. The resultant mixture may optionally undergo subsequent shaping processes.

Shaping processes include, for example, extrusion processes, pelletization, injection molding techniques, blow molding, calendering, spinning, and web melt-blowing.

Alternatively, the inventive compositions are obtainable via a process for stabilizing a polymer, preferably a polyamide, characterized by the following steps
(a) adding the compound of formula (II) to the one or more monomers used for the polymerization of the polyamide in an amount of 0,001 to 1 wt.%, preferably of 0.01 to 0.5 wt.%, more preferably of 0.05 to 0.3 wt.%, based on the total weight of the one or more monomers,
(b) polymerizing the one or more monomers to produce the polyamide.

The monomers are preferably those as described above for the synthesis of polyamides.

The inventive composition may additionally comprise further customary stabilizing agents as listed below.

### 1. Antioxidants

1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methyl-cyclohexyl)-4,6-dimethylphenol, 2,6-dioctadecyl-4-methylphenol, 2,4,6-tricyclohexylphenol, 2,6-di-tert-butyl-4-methoxymethylphenol, nonylphenols which are linear or branched in the side chains, for example 2,6-di-nonyl-4-methylphenol, 2,4-dimethyl-6-(1'-methylundec-1'-yl)-phenol, 2,4-dimethyl-6-(1'-methylheptadec-1'-yl)-phenol, 2,4-dimethyl-6-(1'-methyltridec-1'-yl)-phenol and mixtures thereof.

1.2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol.

1.3. Hydroquinones and alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate, bis-(3,5-di-tert-butyl-4-hydroxyphenyl)-adipate.

1.4. Tocopherols, for example α-tocopherol, β-tocopherol, γ-tocopherol, δ-tocopherol and mixtures thereof (vitamin E).

1.5. Hydroxylated thio-diphenyl ethers, for example 2,2'-thio-bis-(6-tert-butyl-4-methylphenol), 2,2'-thio-bis-(4-octylphenol), 4,4'-thio-bis-(6-tert-butyl-3-methylphenol), 4,4'-thio-bis-(6-tert-butyl-2-methylphenol), 4,4'-thio-bis-(3,6-di-sec-amylphenol), 4,4'-bis-(2, 6-di-methyl-4-hydroxyphenyl)-disulfide.

1.6. Alkylidenebisphenols, for example 2,2'-methylene-bis(6-tert-butyl-4-methylphenol), 2,2'-methylene-bis(6-tert-butyl-4-ethylphenol), 2,2'-methylenebis[4-methyl-6-(α-methylcyclohexyl)phenol], 2,2'-methylene-bis(4-methyl-6-cyclohexylphenol), 2,2'-methylene-bis(6-nonyl-4-methylphenol), 2,2'-methylene-bis(4,6-di-tert-butylphenol), 2,2'-ethylidene-bis(4,6-di-tert-butylphenol), 2,2'-ethylidene-bis(6-tert-butyl-4-isobutylphenol), 2,2'-methylene-bis[6-(α-methylbenzyl)-4-nonylphenol], 2,2'-methylene-bis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methylphenyl)-3-n-dodecylmercaptobutane, ethylene glycol bis[3,3-bis(3'-tert-butyl-4'-hydroxyphenyl)butyrate], bis(3-tert-butyl-4-hydroxy-5-methylphenyl)dicyclopentadiene, bis[2-(3'-tert-butyl-2'-hydroxy-5'-methylbenzyl)-6-tert-butyl-4-methylphenyl]terephthalate, 1,1-bis(3,5-dimethyl-2-hydroxyphenyl)butane, 2,2-bis(3,5-di-tert-butyl-4-hydroxyphenyl)propane, 2,2-bis(5-tert-butyl-4-hydroxy-2-methylphenyl)-4-n-dodecylmercaptobutane, 1,1,5,5-tetra(5-tert-butyl-4-hydroxy-2-methylphenyl)pentane.

1.7. O-, N- and S-benzyl compounds, for example 3,5,3',5'-tetra-tert-butyl-4,4'-dihydroxydibenzyl ether, octadecyl-4-hydroxy-3,5-dimethylbenzylmercaptoacetate, tridecyl-4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl)dithioterephthalate, bis(3,5-di-tert-butyl-4-hydroxybenzyl)sulfide, isooctyl-3,5-di-tert-butyl-4-hydroxybenzylmercaptoacetate.

1.8. Hydroxybenzylated malonates, for example dioctadecyl-2,2-bis(3,5-di-tert-butyl-2-hydroxybenzyl)malonate, di-octadecyl-2-(3-tert-butyl-4-hydroxy-5-methylbenzyl)malonate, didodecylmercaptoethyl-2,2-bis(3,5-di-tert-butyl-4-hydroxybenzyl)malonate, bis[4-(1,1,3,3-tetramethylbutyl)phenyl]-2,2-bis(3,5-di-tert-butyl-4-hydroxybenzyl)malonate.

1.9. Aromatic hydroxybenzyl compounds, for example 1,3,5-tris(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene, 2,4,6-tris(3,5-di-tert-butyl-4-hydroxybenzyl)phenol.

1.10. Triazine compounds, for example 2,4-bis(octylmercapto)-6-(3,5-di-tert-butyl-4-hydroxyanilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris(3,5-di-tert-butyl-4-hydroxyphenoxy)-1,2,3-triazine, 1,3,5-tris(3,5-di-tert-butyl-4-hydroxybenzyl)isocyanurate, 1,3,5-tris(4-tert-butyl-3-hydroxy-2,6-dimethylbenzyl)isocyanurate, 2,4,6-tris(3,5-di-tert-butyl-4-hydroxyphenylethyl)-1,3,5-triazine, 1,3,5-tris(3,5-di-tert-butyl-4-hydroxyphenylpropionyl)hexahydro-1,3,5-triazine, 1,3,5-tris(3,5-dicyclohexyl-4-hydroxybenzyl)isocyanurate.

1.11. Benzylphosphonates, for example dimethyl-2,5-di-tert-butyl-4-hydroxybenzylphosphonate, diethyl-3,5-di-tert-butyl-4-hydroxybenzylphosphonate, dioctadecyl-3,5-di-tert-butyl-4-hydroxybenzylphosphonate, dioctadecyl-5-tert-butyl-4-hydroxy-3-methylbenzylphosphonate, the calcium salt of the monoethyl ester of 3,5-di-tert-butyl-4-hydroxybenzylphosphonic acid.

1.12. Acylaminophenols, for example 4-hydroxylauranilide, 4-hydroxystearanilide, octyl N-(3,5-di-tert-butyl-4-hydroxyphenyl)carbamate.

1.13. Esters of 3-(3,5-di-tert-butyl-4-hydroxyphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)
oxamide, 3-thiaundecanol, 3-thiapentadecanol, trimethylhexanediol, trimethylolpropane, 4-hydroxymethyl-1-phospha-2,6,7 -trioxabicyclo[2.2.2]octane.

1.14. Esters of 3-(5-tert-butyl-4-hydroxy-3-methylphenyl)propionic acid with mono- or polyhydric alcohols, e.g. with methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7 -trioxabicyclo[2.2.2]octane; 3,9-bis[2-{3-(3-tert-butyl-4-hydroxy-5-methylphenyl)propionyloxy}-1,1-dimethylethyl]-2,4,8,10-tetraoxaspiro[5.5]undecane.

1.15. Esters of 3-(3,5-dicyclohexyl-4-hydroxyphenyl)propionic acid with mono- or polyhydric alcohols, e.g. with methanol, ethanol, octanol, octadecanol, 1,6-hexanediol, 1,9-nonanediol,
ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide,3-thiaundecanol, 3-thiapentadecanol, trimethylhexanediol, trimethylolpropane, 4-hydroxymethyl-1-phospha-2,6,7-trioxabicyclo[2.2.2]octane.

1.16. Esters of 3,5-di-tert-butyl-4-hydroxyphenyl acetic acid with mono- or polyhydric alcohols, e.g. with methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phosphat-2,6,7-trioxabicyclo[2.2.2]octane.

1.17. Amides of 3-(3,5-di-tert-butyl-4-hydroxyphenyl)propionic acid e.g. N,N'-bis(3,5-di-tertbutyl-4-hydroxyphenylpropionyl)hexamethylenediamide, N,N'-bis(3,5-di-tert-butyl-4-hydroxyphenylpropionyl)trimethylenediamide, N,N'-bis(3,5-di-tert-butyl-4-hydroxyphenylpropionyl)hydrazide,N,N'-bis[2-(3-[3,5-di-tert-butyl-4-hydroxyphenyl]propionyloxy)ethyl]oxamide (Naugard^{®} XL-1).

1.18. Ascorbic acid (vitamin C)

1.19. Aminic antioxidants, for example N,N'-di-isopropyl-p-phenylenediamine, N,N'-di-sec-butyl-p-phenylenediamine, N,N'-bis(1 ,4-dimethylpentyl)-p-phenylenediamine, N,N'-bis(1-ethyl-3-methylpentyl)-p-phenylenediamine, N,N'-bis(1-methylheptyl)-p-phenylenediamine, N,N'-dicyclohexyl-p-phenylenediamine, N,N'-diphenyl-p-phenylenediamine, N,N'-bis(2-naphthyl)-pphenylenediamine, N-isopropyl-N'-phenyl-p-phenylenediamine, N-(1,3-dimethylbutyl)-N'-phenyl-p-phenylenediamine, N-(1-methylheptyl)-N'-phenyl-p-phenylenediamine, N-cyclohexyl-N'-phenyl-p-phenylenediamine, 4-(p-toluenesulfamoyl)diphenylamine, N,N'-dimethyl-N,N'-disec-butyl-p-phenylenediamine, diphenylamine, N-allyldiphenylamine, 4 isopropoxydiphenylamine, N-phenyl-1-naphthylamine, N-(4-tert-octylphenyl)-1-naphthylamine, N-phenyl-2-naphthylamine, octylated diphenylamine, for example p,p'-di-tert-octyldiphenylamine, 4-n-butylaminophenol, 4-butyrylaminophenol, 4-nonanoylaminophenol, 4-dodecanoylaminophenol, 4-octadecanoylaminophenol, bis(4-methoxyphenyl)amine, 2,6-di-tert-butyl-4-dimethylaminomethylphenol, 2,4'-diaminodiphenylmethane, 4,4'-diaminodiphenylmethane, N,N,N',N'-tetramethyl- 4,4'-diaminodiphenylmethane, 1,2-bis[(2-methylphenyl)amino]ethane, 1,2-bis(phenylamino)propane, (o-tolyl)biguanide, bis[4-(1',3'-dimethylbutyl)phenyl]amine, tert-octylated Nphenyl-1-naphthylamine, a mixture of mono- and dialkylated tert-butyl/tert-octyldiphenylamines, a mixture of mono- and dialkylated nonyldiphenylamines, a mixture of mono- and dialkylated dodecyldiphenylamines, a mixture of mono- and dialkylated isopropyl/isohexyldiphenylamines, a mixture of mono- and dialkylated tert-butyldiphenylamines, 2,3-dihydro-3,3-dimethyl-4H-1,4-benzothiazine, phenothiazine, a mixture of mono- and dialkylated tertbutyl/tert-octylphenothiazines, a mixture of mono- and dialkylated tert-octylphenothiazines, N-allylphenothiazine, N,N,N',N'-tetraphenyl-1,4-diaminobut-2-ene, N,N-bis(2,2,6,6-tetramethylpiperid-4-yl-hexamethylenediamine, bis(2,2,6,6-tetramethylpiperid-4-yl)sebacate, 2,2,6,6-tetramethylpiperidin-4-one, 2,2,6,6-tetramethylpiperidin-4-ol.

2. UV absorbers and light stabilisers

2.1. 2-(2'-Hydroxyphenyl)benzotriazoles, for example 2-(2'-hydroxy-5'-methylphenyl)benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-ditert-butyl-2'-hydroxyphenyl)-5-chlorobenzotriazole, 2-(3'-tert-butyl-2'-hydroxy-5'-methylphenyl)-5-chlorobenzotriazole, 2-(3'-sec-butyl-5'-tert-butyl-2'-hydroxyphenyl)benzotriazole, 2-(2'-hydroxy-4'-octyloxyphenyl)benzotriazole, 2-(3',5'-di-tert-amyl-2'-hydroxyphenyl)benzotriazole, 2-(3',5'-bis(α,α-dimethylbenzyl)-2'-hydroxyphenyl)benzotriazole, 2-(3'-tert-butyl-2'-hydroxy-5'(2-octyloxycarbonylethyl)phenyl)-5-chlorobenzotriazole, 2-(3'-tert-butyl-5'-[2-(2-ethylhexyloxy) carbonylethyl]-2'-hydroxyphenyl)-5-chlorobenzotriazole, 2-(3'-tert-butyl-2'-hydroxy-5'-(2-methoxycarbonylethyl)phenyl)-5-chlorobenzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole, 2-(3'-tert-butyl-2'-hydroxy-5'-(2-isooctyloxycarbonylethyl)phenyl)benzotriazole, 2,2'-methylene-bis-[4-(1,1,3,3-tetramethylbutyl)-6-benzotriazole-2-ylphenol]; the transesterification product of 2-[3'-tert-butyl-5' -(2-methoxycarbonylethyl)-2'-hydroxyphenyl]-2H-benzotriazole with polyethylene glycol 300; [R-CH2CH2'COO-CH2CH2+ where R = 3'-tert-butyl-4'-hydroxy-5'-2H-benzotri-2-azol-2-ylphenyl, 2-[2'-hydroxy-3'-(α,α-dimethylbenzyl)-5'-(1,1,3,3-tetramethylbutyl)phenyl]benzotriazole; 2-[2'-hydroxy-3'-(1,1,3,3 tetramethylbutyl)-5'-(α,α-dimethylbenzyl)phenyl]benzotriazole.

2.2. 2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives.

2.3. Esters of substituted and unsubstituted benzoic acids, for example 4-tert-butyl phenyl salicylate, phenyl salicylate, octylphenyl salicylate, dibenzoyl resorcinol, bis(4-tert-butylbenzoyl)resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate,2-methyl-4,6-di-tert-butylphenyl 3,5-di-tert-butyl-4-hydroxybenzoate.

2.4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate and N-(β-carbomethoxy-β-cyanovinyl)-2-methylindoline.

2.5. Nickel compounds, for example nickel complexes of 2,2'-thiobis[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of the mono-alkyl esters, e.g. the methyl or ethyl ester, of 4-hydroxy-3,5-di-tert-butylbenzylphosphonic acid, nickel complexes of ketoximes, e.g. of 2-hydroxy-4-methylphenylundecylketoxime, nickel complexes of 1-phenyl-4-lauroyl-5-hydroxypyrazole, with or without additional ligands.

2.6. Sterically hindered amines, for example bis(2,2,6,6-tetramethyl-4-piperidyl)sebacate, bis(2,2,6,6-tetramethyl-4-piperidyl)succinate, bis(1,2,2,6,6-pentamethyl-4-piperidyl)sebacate, bis(1-octyloxy-2,2,6,6-tetramethyl-4-piperidyl)sebacate, bis(1,2,2,6,6-pentamethyl-4-piperidyl) n-butyl-3,5-di-tert-butyl-4-hydroxybenzylmalonate, the condensate of 1-(2-hydroxyethyl)- 2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl)nitrilotriacetate, tetrakis(2,2,6,6-tetramethyl-4-piperidyl)-1,2,3,4-butanetetracarboxylate, 1,1'-(1,2-ethanediyl)-bis(3,3,5,5-tetramethylpiperazinone), 4-benzoyl-2,2,6,6-tetramethylpiperidine, 4-stearyloxy-2,2,6,6-tetramethylpiperidine, bis(1,2,2,6,6-pentamethylpiperidyl)-2-n-butyl-2-(2-hydroxy-3,5-di-tert-butylbenzyl)malonate, 3-n-octyl-7,7,9,9-tetramethyl-1,3,8-triazaspiro[4.5]decane-2,4-dione, bis(1-octyloxy-2,2,6,6-tetramethylpiperidyl)sebacate, bis(1-octyloxy-2,2,6,6-tetramethylpiperidyl)succinate, linear or cyclic condensates of N,N'-bis(2,2,6,6-tetramethyl-4-piperidyl)hexamethylenediamine and 4-morpholino-2,6-dichloro-1,3,5-triazine, the condensate of 2-chloro-4,6-bis(4-n-butylamino-2,2,6,6-tetramethylpiperidyl)-1,3,5-triazine and 1,2-bis(3-aminopropylamino)ethane, the condensate of 2-chloro-4,6-di-(4-n-butylamino-1,2,2,6,6-pentamethylpiperidyl)-1,3,5-triazine and 1,2-bis(3-aminopropylamino)ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidine-2,5-dione, 3-dodecyl-1-(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensate of N,N'-bis(2,2,6,6-tetramethyl-4-piperidyl)hexamethylenediamine and 4-cyclohexylamino-2,6-dichloro-1,3,5-triazine, a condensate of 1,2-bis(3-aminopropylamino)ethane and 2,4,6-trichloro-1,3,5-triazine as well as 4-butylamino-2,2,6,6-tetramethylpiperidine (CAS Reg. No. [136504-96-6]); a condensate of 1,6-hexanediamine and 2,4,6-trichloro-1,3,5-triazine as well as N,N-dibutylamine and 4-butylamino-2,2,6,6-tetramethylpiperidine (CAS Reg. No. [192265-64-7]); N-(2,2,6,6-tetramethyl-4-piperidyl)-n-dodecylsuccinimide, N-(1,2,2,6,6-pentamethyl-4-piperidyl)-n-dodecylsuccinimide, 2-undecyl-7,7,9,9-tetramethyl-1-oxa-3,5-diaza-4-oxo-spiro[4,5]decane, a reaction product of 7,7,9,9-tetramethyl-2-cycloundecyl-1-oxa-3,5-diaza-4-oxospiro-[4,5]decane and epichlorohydrin, 1,1-bis(12,2,6,6-pentamethyl-4-piperidyloxycarbonyl)-2-(4-methoxyphenyl)ethene, N,N'-bis-formyl-N,N'-bis(2,2,6,6-tetramethyl-4-piperidyl)hexamethylenediamine, a diester of 4-methoxymethylenemalonic acid with 1,2,2,6,6-pentamethyl-4-hydroxypiperidine, poly[methylpropyl-3-oxy-4-(2,2,6,6-tetramethyl-4-piperidyl)]siloxane, a reaction product of maleic acid anhydride-α-olefin copolymer with 2,2,6,6-tetramethyl-4-aminopiperidine or 1,2,2,6,6-pentamethyl-4-aminopiperidine, N,N'-bis(2,2,6,6-tetramethyl-4-piperidyl)isophthalamide.

2.7. Oxamides, for example 4,4'-dioctyloxyoxanilide, 2,2'-diethoxyoxanilide, 2,2'-dioctyloxy-5,5'-di-tert-butyloxanilide, 2,2'-didodecyloxy-5,5'-di-tert-butyloxanilide, 2-ethoxy-2'-ethyloxanilide, N,N'-bis(3-dimethylaminopropyl)oxamide, 2-ethoxy-5-tert-butyl-2'-ethyloxanilide and its mixture with 2-ethoxy-2'-ethyl-5,4'-di-tert-butyloxanilide, mixtures of o- and p-methoxy-disubstituted oxanilides and mixtures of o- and p-ethoxy-disubstituted oxanilides.

2.8. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethyl)-1,3,5-triazine, 2-[2-hydroxy-4-(2-hydroxy-3-octyloxypropyloxy) phenyl]-4,6-bis(2 ,4-dimethyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3 dodecyloxypropoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxypropoxy)phenyl]-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2 hydroxypropyloxy]phenyl}-4,6-bis(2,4-dimethylphenyl)-1,3,5-triazine.

3. Metal deactivators, for example N,N'-diphenyloxamide, N-salicylal-N'-salicyloyl hydrazine, N,N'-bis(salicyloyl)hydrazine, N,N'-bis(3,5-di-tert-butyl-4-hydroxyphenylpropionyl) hydrazine, 3-salicyloylamino-1,2,4-triazole, bis(benzylidene)oxalyl dihydrazide, oxanilide, isophthaloyl dihydrazide, sebacoyl bisphenylhydrazide, N,N'-diacetyladipoyl dihydrazide, N,N'-bis(salicyloyl)oxalyl-dihydrazide, N,N'-bis(salicyloyl)thiopropionyl dihydrazide.

4. Phosphites and phosphonites, for example triphenyl phosphite, diphenylalkyl phosphites, phenyldialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl)pentaerythritol diphosphite, bis(2,4-dicumylphenyl)pentaerythritol diphosphite, bis(2,6-di-tert-butyl-4-methylphenyl)pentaerythritol diphosphite, diisodecyloxypentaerythritol diphosphite, bis(2,4-di-tert-butyl-6-methylphenyl)pentaerythritol diphosphite, bis(2,4,6-tris(tert-butylphenyl))pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenz[d,g]-1,3,2-dioxaphosphocin, bis(2,4-di-tert-butyl-6-methylphenyl)methyl phosphite, bis(2,4-di-tert-butyl-6-methylphenyl)ethyl phosphite, 6-fluoro-2,4,8,10-tetra-tert-butyl-12H-methyl-dibenz[d,g]-1,3,2-dioxaphosphocin, 2,2',2"-nitrilo[triethyltris(3,3',5,5'-tetra-tert-butyl-1,1'-biphenyl-2,2'-diyl)phosphite], 2-ethylhexyl(3,3',5,5'-tetra-tert-butyl-1,1 '-biphenyl-2,2'-diyl)phosphite, 5-butyl-5-ethyl-2-(2,4,6-tri-tert-butylphenoxy)-1,3,2-dioxaphosphirane.

5. Hydroxylamines, for example N,N-dibenzylhydroxylamine, N,N-diethylhydroxylamine, N,N-dioctylhydroxylamine, N,N-dilaurylhydroxylamine, N,N-ditetradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.

6. Nitrones, for example N-benzyl-α-phenylnitrone, N-ethyl-α-methylnitrone, N-octyl-α-heptylnitrone, N-lauryl-α-undecylnitrone, N-tetradecyl-α-tridecylnitrone, N-hexadecyl-α-pentadecylnitrone, N-octadecyl-α-heptadecylnitrone, N-hexadecyl-α-heptadecylnitrone, N-octadecyl-α-pentadecylnitrone, N-heptadecyl-α-heptadecylnitrone, N-octadecyl-α-hexadecylnitrone, nitrone derived from N,N-dialkylhydroxylamine derived from hydrogenated tallow amine.

7. Thiosynergists, for example dilauryl thiodipropionate, distearyl thiodipropionate or dioctadecyl disulphide.

8. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulphide, pentaerythritol tetrakis(β-dodecylmercapto)propionate.

9. Polyamide stabilisers, for example copper salts in combination with iodides and/or phosphorus compounds and salts of divalent manganese.

10. Basic co-stabilisers, for example melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids, for example calcium stearate, zinc stearate, magnesium behenate, magnesium stearate, sodium ricinoleate and potassium palmitate, antimony pyrocatecholate or zinc pyrocatecholate.

11. Conventional nucleating agents, for example inorganic substances, such as talcum, metal oxides, such as titanium dioxide or magnesium oxide, phosphates, carbonates or sulfates of, preferably, alkaline earth metals; organic compounds, such as mono- or polycarboxylic acids and the salts thereof, e.g. 4-tert-butylbenzoic acid, adipic acid, diphenylacetic acid, sodium succinate or sodium benzoate; polymeric compounds, such as ionic copolymers (ionomers). Especially preferred are 1,3:2,4-bis(3',4'-dimethylbenzylidene) sorbitol, 1,3:2,4-di(paramethyldibenzylidene)sorbitol, and 1,3:2,4-di(benzylidene)sorbitol.

12. Other additives, for example plasticisers, lubricants, rheology additives, catalysts, flow control agents, optical brighteners, flameproofing agents, antistatic agents and blowing agents.

13. Benzofuranones and indolinones, for example those disclosed in US-A-4,325,863; US-A-4,338,244; US-A-5,175,312; US-A-5,216,052; US-A-5,252,643; DE-A-4316611; DE-A-4316622; DE-A-4316876; EP-A-0589839 or EP-A-0591102 or 3-[4-(2-acetoxyethoxy)phenyl]-5,7-di-tert-butylbenzofuran-2-one, 5,7-di-tert-butyl-3-[4-(2-stearoyloxyethoxy)phenyl]benzofuran-2-one, 3,3'-bis[5,7-di-tert-butyl-3-(4-[2-hydroxyethoxy]phenyl)benzofuran-2-one], 5,7-di-tert-butyl-3-(4-ethoxyphenyl)benzofuran-2-one, 3-(4-acetoxy-3,5-dimethylphenyl)-5,7-di-tert-butylbenzofuran-2-one, 3-(3,5-dimethyl-4-pivaloyloxyphenyl)-5,7-di-tert-butylbenzofu ran-2-one, 3-(3,4-dimethylphenyl)-5,7-di-tert-butylbenzofuran-2-one, 3-(2,3-dimethylphenyl)-5,7-di-tert-butylbenzofuran-2-one.

The present invention is further illustrated by the following examples and claims. The examples show the preferred embodiments of the invention.

### Examples

Unless otherwise stated all values given in wt% are directed to % by weight and all ratio given are based on weight ratio.

The following substances were used for the Examples described below. The compounds of formula (II) were produced as described in example 1:

**Table 1: Substances**

| **Abbreviation** | **Product Name** | **Producer supplier** | **Chemical class** |
|---|---|---|---|
| **Polymers** | | | |
| PA 6 | PA 6 UBE 1022B, Lot: T506B61, RV 3.36 @ 96% H2SO4, Cone. 1% | Ube | Polyamide 6 |
| PP 2602 | Licocene^{®} PP 2602 | Clariant SE | LMW PP based Copolymer (not pre-stabilized) |
| | | | CAS No.: 9010-79-1 |
| PP-R | | Sibur | HMW-PP based random Copolymer, which is polymerized via Novolen process (not pre-stabilized) |
| | | | CAS No.: 9010-79-1 |

| **HALS-Stabilizers** | | | |
|---|---|---|---|
| S1 | Dibutyl-N,N'-bis(2,2,6,6-tetramethyl-4-piperidinyl)-1,3-benzenedicarboxamide | Experimental Clariant product | LMW-HALS* based on n-butyl-TAD and isophthalic acid |
| S2 | Dibutyl-N,N'-bis(2,2,6,6-tetramethyl-4-piperidinyl)-1,4-benzenedicarboxamide | Experimental Clariant product | LMW-HALS* based on n-butyl-TAD and terephthalic acid |
| NS | Nylostab^{®} S-EED | Clariant SE | LMW-HALS* |
| N30 | Hostavin^{®} N 30 | Clariant SE | HMW-HALS** |
| N28 | Hostavin^{®} N 28 | Clariant SE | LMW-HALS* |
| T770 | Tinuvin^{®} 770 | BASF AG | LMW-HALS* |
| C944 | Chimassorb^{®} 944 | BASF AG | HMW-HALS** |

| **sterically hindered phenolic primary antioxidants** | | | |
|---|---|---|---|
| B215 | Irganox^{®} B 215 | BASF AG | Blend of 66% Irgafos^{®} 168 und 33% Irganox^{®} 1010 |
| | Irganox^{®} 1010 | BASF AG | pentaerythritol tetrakis[3-[3,5-di-tert-butyl-4-hydroxyphenyl]propionate |
| | Irgafos^{®} 168 | BASF AG | Tris (2,4-di-tert-butylphenyl) phosphite |

| **Further Additives** | | | |
|---|---|---|---|
| CaSt | | | Calcium Stearate lubricant |

| | | | |
|---|---|---|---|
| * LMW HALS = low molecular weight HALS ** oligomeric HMW HALS = oligomeric high molecular weight HALS | | | |

**Table 2: Methods for substance characterization and evaluation of substance performance**

| | Method |
|---|---|
| Solubility [g/cm3] | Described in experimental part |
| Relative viscosity | ηᵣₑₗ = η/ηₛ |
| | ratio of the viscosity of a solution (η) to the viscosity of the solvent used (ηₛ) |
| Melting point Tm [°C]; 1st and 2nd heating | DIN EN ISO 11357-3 |
| 1^{st}: from 50 to 200°C at 10 K/min, then 3 min at 200°C. | |
| Cooling: from 200-50°C at 10K/min, then 3 min at 50°C | |
| 2^{nd}: from 50 to 200°C at 10 K/min | |
| Glass transition temperature Tg [°C] | DIN EN ISO 11357-2 |
| Melt flow rate (MFR) | ISO 1133B |
| Enthalpy of fusion (EF) [J/g] | DIN EN ISO 11357-1 |
| Detectable agglomerates/defects by visual observation | Internal Clariant method: |
| | Number of particles are calculated by sum of 3 positions in one plate with measurement area of 1*1 cm² of each position. |
| Thermogravimetric analysis (TGA)[% by wt.] from 30 to 550°C at 10 K/min | DIN 51006 |
| Measurement of loss of mass on attainment of 240°C and measurement of temperature when 10 wt % are lost | |
| Discoloration ΔE | CIE 1964 / observer 10 degree / Illuminant D 65 (with Spectrophotometer) |
| calculated from CIEL*a*b* color values as difference between the displayed color after exposure and the original color of the polymer samples. | |
| Yellowness-Index (YI) | ASTM E 313 - 20 |
| 2θ-angle and Intensity (Int) from XRD powder diffractogram (all 2θ-angle values have an uncertainty of +/-0,2°) | samples were measured on a STOE STADI P powder diffractometer with a Ge (111) monochromator and a position-sensitive detector (linear PSD) with Cu-Kα₁ radiation (wavelength 1.5406 Angström). The measurements were carried out between polymer films in transmission geometry over an 2θ angle range of 4° to 40°. The measurement time was approx. 1 h. The samples were rotated during the measurement. The data acquisition and analysis was carried out with STOE WinXPOW program suite. |
| | Intensities from visual estimation are classified as follows: ss = very strong, s = strong, m = moderate (medium), w = weak, vw = very weak, br = broad or splitted peak |
| Xenon Weathering Exposure Test | ISO 4892-2 wet/dry |

### A.) Preparation and XRD-characterization of inventive stabilizers (S)

### Example 1: Preparation of N1,N3-dibutyl-N1,N3-bis(2,2,6,6-tetramethyl-4-piperidyl)benzene-dicarboxamide stabilizers (S)

### Example 1(A) (Preparation of S1) (Alcohol process):

A mixture of 600 g of n-propanol or 2-propanol, 300 g of deionized water and 212 g (1.0 mol) of n-butyl-TAD was charged into a reactor under nitrogen atmosphere. Afterwards, 108 g (0.53 mol) of isophthaloyl dichloride was added continuously under stirring. Then, 92 g (1.15 mol) of a 50 wt-% aqueous NaOH solution was added under continuous stirring at ambient conditions. Subsequently, it was stirred for 6 hours under reflux. The aqueous phase was separated off. Then, the organic solvent was replaced with water by continuous distillation of propanol and addition of water. Then, an organic solvent was added. Afterwards, the water phase was separated off and the organic phase was dried until completion. The product was obtained as a colourless to yellow clear melt 253,0 g (~91%).

### Example 1(B) (Preparation of S1) (Cyclohexane process):

A mixture of 800 g of cyclohexane, 300 g of deionized water and 212 g (1.0 mol) of n-butyl-TAD was charged into a reactor under nitrogen atmosphere. Then, 92 g (1.15 mol) of a 50 wt-% aqueous NaOH solution was added under continuous stirring at ambient conditions. Afterwards, 108 g (0.53 mol) of isophthaloyl dichloride was added continuously under stirring. Subsequently, it was stirred for 3-6 hours under reflux. The aqueous phase was separated off and the organic phase was washed 2 times with 300 g of deionized water. The cyclohexane phase was cooled to room temperature under stirring, the precipitated solid was filtered off, washed with cyclohexane, and dried under vacuum to constant weight. The product was obtained as a white to off-white powder 205,2 g (-74%).

### Example 1(C) (Preparation of S1):

A mixture of 600 g of xylene and 212 g (1.0 mol) of n-butyl-TAD was charged into a reactor under nitrogen atmosphere. Then, 106,2 g (1.05 mol) triethylamine was added under continuous stirring at ambient conditions. Afterwards, 108 g (0.53 mol) of isophthaloyl dichloride was added continuously under stirring. Subsequently, it was stirred for 3-6 hours under reflux. Then, the organic phase was washed 3 times with 300 g of deionized water. The organic phase was evaporated and dried. The product was obtained as an orange-yellow to brown melt 146,8 g (-36%).

### Example 1(D) (Preparation of S2)

A mixture of 600 g of n-propanol or 2-propanol, 300 g of deionized water and 212 g (1.0 mol) of n-butyl-TAD was charged into a reactor under nitrogen atmosphere. Afterwards, 108 g (0.53 mol) of terephthaloyl dichloride was added continuously under stirring. Then, 92 g (1.15 mol) of a 50 wt-% aqueous NaOH solution was added under continuous stirring at ambient conditions. Subsequently, it was stirred for 6 hours under reflux. The aqueous phase was separated off. Then, the organic solvent was replaced with water by continuous distillation of propanol and addition of water. Then the precipitant was filtered and washed with deionized water. The precipitant was dried to completion. The product was obtained as a white to off-white powder 244,8 g (~88 %).

### Example 1(E) (Preparation of S1) (Aromatic process)

A mixture of 700 g of xylene, 338 g of deionized water and 212 g (1.0 mol) of n-butyl-TAD was charged into a reactor under nitrogen atmosphere. Afterwards, 108 g (0.53 mol) of isophthaloyl dichloride was added continuously under stirring. Then, 92 g (1.15 mol) of a 50 wt-% aqueous NaOH solution was added under continuous stirring at ambient conditions. Subsequently, it was stirred for 6 hours under reflux. The aqueous phase was separated off and the organic phase was washed 2 times with 300 g of deionized water each time. The organic phase was evaporated and dried. The product was obtained as a colourless to yellow clear melt 249,5 g (-90%).

### Example 2: Preparation and XRD-characterization of different amorphous and crystalline phases of S1

Surprisingly, it was found that S1 can exist in an entirely amorphous state and in different crystalline structures. The different crystal phases of the inventive stabilizer S1 were prepared using one of the above-mentioned techniques (such as Example 1(B)) and annealing it in the following ways. The purity of the inventive stabilizer S1 was analyzed to be 99,4 area-% and was the same for all phase preparations.

### Example 2(A): S1 α-phase:

S1 was molten in a round bottomed flask for at least 60 minutes. Then, the melt was allowed to cool to room temperature. Subsequently, liquid nitrogen was poured into the round bottomed flask and was allowed to stand for 30 minutes until all the nitrogen evaporated. Finally, the sample was allowed to warm up to ambient temperatures and was subsequentially crushed and grinded or milled to a powder.

The complete list of reflections for the α-phase is shown in Table 3. The reflection intensities are derived from Figure 2.

### Example 2(B): S1 β-phase:

S1 was molten in a round bottomed flask for at least 60 minutes. Then, it was cooled to ambient conditions and slowly heated to 104°C. Then, the melt was annealed at 104°C until the melt was crystallized completely.

The complete list of reflections for the β-phase is shown in Table 3. The reflection intensities are derived from Figure 3.

### Example 2(C): S1 - mixture of S1 γ- and S1 α-phase:

In order to obtain a mixture of α- and γ phase the above-mentioned preparation example 1(B) (crystallization from cyclohexane) was followed.

The complete list of reflections for the mixture of α- and γ-phase is shown in Table 3. The reflection intensities are derived from Figure 4.

### Example 2(D): S1 - amorphous

S1 was molten in a round bottomed flask for at least 60 minutes and pastillated using a heated dropping funnel and short pastillation belt. For the XRD measurements small round plates were created having 0.5 - 1 cm in diameter and 0.5 - 1 mm in thickness using a heated dropping funnel and silicone moulds with the respective geometries. Depending on sample preparation, minor reflections of the α-, β- and/or γ-phase can be found in the XRD diffractogram (Figure 1).

The samples from Examples 2(A) - 2(D) were determined to have a purity of 99,4 area-% measured in GC.

The polymorphic phases from Examples 2(A) - 2(D) were analyzed by XRD measurements.

In the X-ray diffractogram, the following reflections were identified for different crystal phases of the product. The characteristic reflections (also called "signals" or "peaks") of the α-phase (Figure 2), the β-phase (Figure 3) and the mixture of the α- and γ-phase are given in Table 3.

The Intensity ("Int") denotes the intensities from visual estimation as follows: ss = very strong, s = strong, m = moderate (medium), w = weak, vw = very weak, br = broad or splitted peak. In the mixture of γ- and α-phases, the column "Phase" describes a visual assignment of all peaks to the γ- and α -phase, or as a superposition of peaks of both phases ("α+γ"). Int(α+γ) denotes the visually estimated intensities of the peaks as observed in the mixture of the γ-phase and α -phase. Int(γ) denotes the visually estimated intensities of the signals of the pure γ-phase as obtained by visual extraction from the α/γ -mixture. The expression "n.d." denotes that the intensity of the corresponding reflection of the γ -phase could not be determined due to peak overlap with the α -phase.

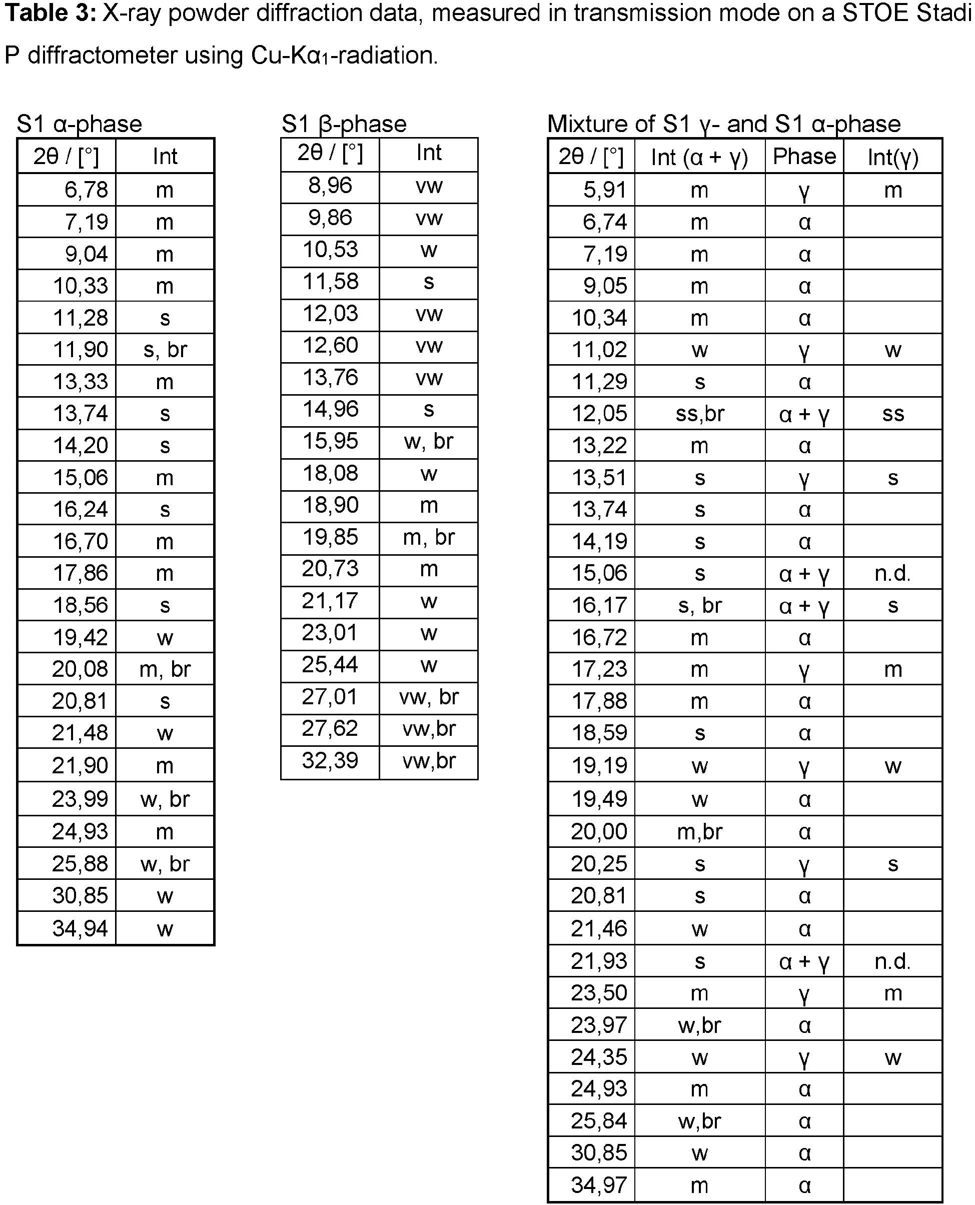

2θ denotes the peak positions given as diffraction angle in 2θ in degrees. All 2θ values have an uncertainty of +/- 0.2° in 2θ.

The amorphous phase is characterized by the characteristic amorphous scattering pattern in the X-ray powder diffractogram, measured with Cu-Kα1 radiation at a 2θ angle between 3° and 25° and the absence of distinct reflexes. Depending on sample preparation, low intensity, distinct reflexes of the α-, β- and γ-phase can be present.

### Example 3: Thermal data of the inventive stabilizers and the reference stabilizer NS

The thermal data of inventive and comparative stabilizers NS, S1 and S2 was measured with DSC and TGA.

**Table 4: Thermal data of inventive and comparative stabilizers S1, S2 and NS:**

| Stabilizer | **S1** | **S2** | **NS** |
|---|---|---|---|
| Tm or range of Tm's from 1^{st} heating curve [°C] | 119-130 | 160 | 274 |
| 10% decomp. [°C] (TGA) | 305 | 300 | 320 |

S1 and S2 have, in comparison to NS, a significantly reduced melting point (range) due to the butylation of the amid functionalities. Surprisingly, the melting point of S1 is significantly lower due to the 1,3-substitution (meta/isophthal) of the BCA building block over the 1,4-substitution (para/terephthal) in S2, while the 10% decomposition temperature is only slightly affected by the butylation.

**Table 4a: Thermal data of the amorphous phase and the different crystal structures of S1**

| Stabilizer | melting point [°C] 1st heating curve | glass transition temp. [°C] 1st heating curve | melting point [°C] 2nd heating curve | glass transition temp.[°C] 2nd heating curve |
|---|---|---|---|---|
| **S1-amorph** | - | 26-32°C | * | 26-32°C |
| **S1-α** | 119 | - | * | 26-32°C |
| **S1-β** | 130 | - | * | 26-32°C |
| **S1-αγ** | 123 | - | * | 26-32°C |

| | | | | |
|---|---|---|---|---|
| * there is no melting point Tm detectable in the 2^{nd} heating curve | | | | |

Surprisingly, it was found that the different crystal structures of S1 have different distinct melting points. They are much higher than the glass transition temperature of the amorphous phase.

### B.) Application tests in polyamides

### B.1) Solubility Tests in selected monomers

### Example 4: Solubility tests in PA 6 and PA 66 monomers

The solubility of comparative stabilizer NS and the inventive stabilizers S1 and S2 was determined in ε-caprolactam and hexamethylene diamine to evaluate their suitability for upstream processing during polymerization of PA 6 (ε-caprolactam) and PA 66 (hexamethylene diamine) as these substances are the monomers for the production of PA 6 (ε-Caprolactam) and PA 66. Further the temperature dependency of the solubility was evaluated.

100 g of one of the monomers as listed in Table 1 were filled in a round bottom flask equipped with a magnetic stirrer, reflux condenser and internal thermometer, and heated to the specified temperature (60°C, 80°C and 90°C).

**Table 5: Solubility Tests for upstream application - Temperature dependency**

| Temperature | Substance in Solvent [g/100g] | | | | | |
|---|---|---|---|---|---|---|
| | ε-caprolactam (PA 6 monomers) | | | hexamethylene diamine (one of the PA 66 monomers) | | |
| | S1 | NS | S2 | S1 | NS | S2 |
| 60 °C | Not applicable | | | 34,7 | 5,5 | 3,2 |
| 80°C | | | | 57 | 6,5 | 8,8 |
| 90 °C | 42,4 | 14,4 | 12,0 | > 100 | 9,0 | 14,5 |

The solubility of S1 in ε-Caprolactam is three times higher than that of NS, the solubility of S1 in hexamethylene diamine is six to nine times higher than that of NS. Therefore, S1 is more suitable for the upstream incorporation of the stabilizer in the polyamide as NS, as S1 has a much higher solubility and dissolves faster in the monomers used for the PA 6 or PA 66 polymerization. Thus, it shows that the introduction of the butyl group increases the solubility in both monomers. Surprisingly, the data shows that only the meta position (isophthalic S1) increases the solubility significantly, whereas the para position (terephthalic S2) has either no impact (Caprolactam) or only little impact (HMDA) on solubility.

**Table 6: Solubility ([g/100 ml]) of inventive and comparative stabilizer substances in various solvents at different temperatures.**

| **Solubility [g /100 mL]** | | **NS** | **S1** | **S2** |
|---|---|---|---|---|
| n-Butyl acetate | RT | 0,7 | 21,9 | 0,8 |
| | 80°C | 0,7 | > 100 | 7,2 |
| Cyclohexane | RT | 2,4 | 2,4 | 0,4 |
| | 80 °C | 3,2 | 22,7 | 2 |
| Ethyl methyl ketone (MEK) | RT | 0,1 | 60 | 1,5 |
| | 50°C | 1,0 | > 100 | 9,0 |
| Methanol | RT | 6,0 | > 100 | 65,6 |
| | 50°C | 10,7 | > 100 | 72,0 |
| 1-Methoxy-2-propyl acetate (MPA) | RT | 0,2 | 10,4 | 0,4 |
| | 80°C | 0,4 | 90,0 | 2,4 |
| Methyl isobutyl ketone (MIBK) | RT | 0,4 | 32,4 | 0,8 |
| | 50 °C | 0,8 | 71,2 | 4,0 |
| Dihydrolevoglucosenone (Cyrene^{™}) | RT | 0,1 | 23,6 | 0,4 |
| | 50 °C | 0,4 | 52,0 | 0,8 |
| Xylene | RT | 0,1 | 41,1 | 0,6 |
| | 80°C | 0,2 | > 100 | 12,4 |

S1 shows superior solubility compared to NS and S2 when dissolved in common solvents for coating systems including xylene, MPA, MEK, MIBK, and n-butylacetate. This indicates that S1 has good compatibility with solvents-, which are used for coating systems based on resins, such as 2K-PU, 1K-acrylics, epoxides, alkyds, and polyester resins. The increased solubility of S1 is attributed not only to the butyl groups, but surprisingly also to the 1,3-substitution (meta/isophthal) of the BCA building block, which is significantly increasing the solubility compared to the 1,4-substitution (para/terephthal) of S2.

### B.2) Application tests in PA 6

### Example 5: Preparation of the PA 6 compound pellets comprising the inventive or reference stabilizer

The following pellets of the PA 6 compounds were prepared using PA 6 polyamide (PA 6 UBE 1022B) and either S1 or NS:
15 - 20 kg of PA 6 pellets were grinded in a pallmann grinder to pulverize the pellets. 2 kg of this powder was put into a Santos mixer with aluminium flat beater and the stabilizer was added in the amount shown in Table 7 above and stirred for premixing.

**Table 7: Stabilizer/polyamide compositions (all amounts given in % by weight referring to the total amount of polyamide and stabilizer)**

| **Formulation** | **Composition** |
|---|---|
| F1@240°C | PA 6 |
| F2@240°C | PA 6 + 0.3% S1 |
| F3@240°C | PA 6 + 0.5% S1 |
| F4@240°C | PA 6 + 0.3% NS |
| F5@240°C | PA 6 + 0.5% NS |
| F6@280°C | PA 6 + 0.3% NS |
| F7@280°C | PA 6 + 0.5% NS |

The pre-mixed stabilizer/PA 6 formulation was dried at 80°C in powder form and cooled down for 5-7 hrs in the vacuum oven under N₂ gas and then further compounded.

The compounds were pre extruded at 240°C (S1, NS) or 280°C (NS) in a Collin single screw extruder (25 x 25D) with water bath and pelletized.

The pellets were dried again in the vacuum oven at 80 °C under N₂ gas for a minimum of 12 hours until the moisture content is less than 0,05%.

### Example 6: Stabilizer distribution in injection molded PA 6 plates

The pellets from example 6 above were processed in an Arburg injection molding machine at 250°C to form the injected plates with the following dimensions of the test specimen: plate size 10x10 cm²; plate thickness: 1 mm.

**Table 8: Number of detectable particles in injection molded plates**

| No. | Formulation | Number of particles (Classification by size) | | | | |
|---|---|---|---|---|---|---|
| | | < 100 µm | 100 µm - 200 µm | 200 µm - 300 µm | 300 µm - 400 µm | > 400 µm |
| Ex.1 | F1@240°C | 264 | | | | |
| Ex.2 | F2@240°C | 259 | | | | |
| Ex.3 | F4@240°C | 364 | 4 | 6 | 1 | - |
| Ex.4 | F6@280°C | 418 | | | | |

| | | | | | | |
|---|---|---|---|---|---|---|
| Ex. 1 and 2 produce homogeneous plates with 25% less irregularities under 100 µm and no irregularities with particle size of more than 100 µm. Comparative Ex. 3 shows much more irregularities at low particle sizes below 100 µm and even large irregularities with up to 400 µm, because of the reduced compatibility of NS at 240 °C. It can be derived that unmelt NS was observed in the injected plate when compounding at 240 °C. Therefore, higher processing temperatures for NS are necessary to produce homogeneous formulations with well distributed stabilizers, which cause more unwanted thermal-oxidative stress to the formulation. Ex 4, which evaluated a PA 6 / NS formulation at 280 °C shows, that the large irregularities caused by NS disappear, but the plate still shows more small irregularities than Ex. 1 and 2 for low particle sizes below 100 µm. | | | | | | |

### Example 7: Evaluation of Yellowness Index and discoloration ΔE of PA 6 plates after Xenon Weathering Exposure Test (WOM)

The pellets from example 6 above were processed in an Arburg injection molding machine at 250 °C to form the injected plates with the following dimensions of the test specimen: plate size 10x10 cm²; plate thickness: 1 mm.

Surface cracking resistance, plate appearance, yellowing (YI) and discoloration (ΔE), caused by the accelerated artificial Xenon Weathering Exposure Test (WOM) were determined for the samples listed in Tables 9a and 9b using the testing methods as described in Table 1 above. The test results are summarized in Tables 9a and 9b.

**Table 9a: Yellowness Index (YI) after WOM exposure**

| | WOM time | Initial | 500 hrs | 1000 hrs | 1500 hrs | 2000 hrs | 2500 hrs | 3000 hrs | 3500 hrs | 4000 hrs |
|---|---|---|---|---|---|---|---|---|---|---|
| No. | Formulations | | | | | | | | | |
| Ex. 5 | F-1@240 | 1,90 | 3,29 | 3,61 | Sample disintegrated | | | | | |
| Ex. 6 | F-2@240 | 3,09 | 5,29 | 5,30 | 5,09 | 4,845 | 4,945 | 5 | 5,205 | 5,875 |
| Ex. 7 | F-6@280 | 3,87 | 7,44 | 7,42 | 7,49 | 7,225 | 7,27 | 7,11 | 7,245 | 7,69 |

**Table 9b: ΔE after WOM exposure**

| | WOM time | 500 hrs | 1000 hrs | 1500 hrs | 2000 hrs | 2500 hrs | 3000 hrs | 3500 hrs | 4000 hrs |
|---|---|---|---|---|---|---|---|---|---|
| No. | Formulations | | | | | | | | |
| Ex. 5 | F-1@240 | 0,9 | 1,0 | Sample disintegrated | | | | | |
| Ex. 6 | F-2@240 | 1,3 | 1,3 | 1,1 | 1,0 | 1,1 | 1,1 | 1,2 | 1,6 |
| Ex. 7 | F-6@280 | 2,1 | 2,0 | 2,1 | 2,0 | 2,0 | 1,9 | 2,0 | 2,2 |

Polymer stabilization is crucial to avoid sample disintegration to weathering conditions due to polymer degradation. The test plate which was not stabilized (Ex. 5), disintegrated after 1000 hours of WOM exposure, so its lifetime is very limited. Plates stabilized with NS are much more durable, but yellow and discolor strongly over time. S1 stabilized samples yellow after 500 hours (Ex. 6 YI = 5,29) less than the samples stabilized with NS (Ex. 7 YI = 7,44) and have no significant color change afterwards until sample disintegration. Thus, the color retention of Ex. 6 is better than the color retention of Ex. 7. The same is observable for ΔE.

### Example 8: Evaluation of Yellowness Index and discoloration ΔE of PA 6 pellets after short term oven storage at 210 °C.

The pellets from Example 6 were stored in an oven at 210 °C for 30 minutes to simulate severe thermal ageing conditions. The yellowing index YI and the discoloration ΔE of the PA 6 pellets was measured every 5 minutes. The results are summarized in Tables 10a and 10b.

**Table 10a: Yellowness Index (YI) after x minutes of oven storage at 210°C**

| No. | Formulations | Initial | 5 min | 10 min | 20 min | 30 min |
|---|---|---|---|---|---|---|
| Ex. 8 | F-1@240 | 3,89 | 4,27 | 4,31 | 5,38 | 5,72 |
| Ex. 9 | F-2@240 | 5,88 | 6,13 | 6,07 | 7,19 | 7,84 |
| Ex. 10 | F-6@280 | 8,44 | 8, 76 | 9,02 | 10,92 | 11,705 |

Table 10b shows the stabilizing effect on discoloration of PA 6 pellets after 30 minutes of oven exposure at 210°C.

**Table 10b: ΔE after 30 minutes of oven storage at 210°C**

| | | ΔE | | | |
|---|---|---|---|---|---|
| No. | Formulation | 5 min | 10 min | 20 min | 30 min |
| Ex. 8 | PA 6 | 0,23 | 0,2 | 0,78 | 1,12 |
| Ex. 9 | PA 6 + 0.3% S1 | 0,17 | 0,15 | 0,71 | 1 |
| Ex. 10 | PA 6 + 0.3% NS | 0,26 | 0,38 | 1,31 | 1,7 |

The color retention of PA 6 pellets stabilized with S1 (Ex. 9) is lower after thermal treatment at 210°C than the color retention of the unstabilized polymer (Ex. 8) or the polymer stabilized with NS (Ex. 10). Hence, the inventive stabilizer can be used for long-term heat stabilization.

### C) Application Tests in polypropylene

### Example 9: Thermal stability and optical color impression of unstabilized and stabilized Licocene PP 2602 (low molecular weight PP)

Licocene PP 2602 was heated to 170°C in a large test tube together with the stabilizers listed below at concentrations of 0.1% and 0.25% until the Licocene PP 2602 had melted. The mixtures were then homogenized with a propeller stirrer for 15 minutes. Subsequently, these mixtures were left in a metal heating block at 170°C for 7 days or 168 h under air. After 24 h, 48 h, 72 h, 96 h and 168 h, samples were taken and the dynamic viscosity at 170°C (Table 11a) and color (Table 11b) were determined.

**Table 11a: dynamic viscosity of Licocene PP 2602 with 0,1 wt% or 0,25 wt% stabilizers**

| | time@ 170°C | Amount of Stabilizer [%] | | 0h | 24h | 48h | 72h | 96h | 168h |
|---|---|---|---|---|---|---|---|---|---|
| No. | | | | | | | | | |
| Ex. 11 | | w/o S | | 5466 | 5333 | 4405 | 3066 | 2232 | 898 |
| Ex. 12 | | B215 | 0,1 | 6230 | 6197 | 6001 | 4296 | 3067 | 1444 |
| Ex. 13 | | | 0,25 | 6021 | 6101 | 6090 | 5910 | 5615 | 3805 |
| Ex. 14 | | N30 | 0,1 | 6241 | 6032 | 5759 | 4899 | 4323 | 3029 |
| Ex. 15 | | | 0,25 | 6087 | 5974 | 5759 | 5209 | 4750 | 3805 |
| Ex. 16 | | N28 | 0,1 | 6171 | 6072 | 5963 | 5022 | 4037 | 2691 |
| Ex. 17 | | | 0,25 | 6044 | 5981 | 5892 | 5514 | 5092 | 3764 |
| Ex. 18 | | T770 | 0,1 | 6279 | 6024 | 5947 | 5112 | 4493 | 2766 |
| Ex. 16 | | | 0,25 | 6082 | 5835 | 5789 | 5899 | 5367 | 4303 |
| Ex. 17 | | S1 | 0,1 | 6185 | 6122 | 6195 | 5617 | 5157 | 3913 |
| Ex. 18 | | | 0,25 | 6086 | 6064 | 6149 | 5675 | 5658 | 5002 |
| Ex. 19 | | S2 | 0,1 | 6173 | 6071 | 6235 | 5554 | 4935 | 3646 |
| Ex. 20 | | | 0,25 | 6101 | 5959 | 5938 | 5643 | 5428 | 4544 |

The inventive stabilizers can be used for long term heat stabilization in Licocene PP 2602 because of their low melting point. They especially show better heat stabilizing effects than other HALS stabilizers and the classical phenol-based stabilizer package B215. Further, S1 is more efficient than the classical B215, with 0.1 wt. % additvation of S1 the same stabilization performance can be achieved as with B215 at 0.25wt. % additivation.

The optical color impression was assessed by a test person who classified the optical color impression according to the following scale: 1 ≙ white, 2 ≙ offwhite, 3 ≙ slightly yellowish, 4 ≙ yellowish, 5 ≙ yellowish-brown, 6 ≙ brown

**Table 11b: optical color impression of Licocene PP 2602 with 0,1 wt% and 2,5 wt% stabilizers**

| No. | Amount of Stabilizer | | 0h | 24h | 48h | 72h | 96h | 168h |
|---|---|---|---|---|---|---|---|---|
| Ex. 11 | w/o S | | 1 | 1 | 3 | 5 | 6 | 6 |
| Ex. 12 | B215 | 0,1 | 1 | 1 | 2 | 4 | 5 | 6 |
| Ex. 13 | | 0,25 | 1 | 1 | 1 | 1 | 2 | 4 |
| Ex. 14 | N30 | 0,1 | 1 | 1 | 2 | 3 | 4 | 5 |
| Ex. 15 | | 0,25 | 1 | 1 | 1 | 1 | 3 | 4 |
| Ex. 16 | N28 | 0,1 | 1 | 1 | 1 | 2 | 4 | 6 |
| Ex. 17 | | 0,25 | 1 | 1 | 1 | 1 | 2 | 4 |
| Ex. 18 | T770 | 0,1 | 1 | 1 | 1 | 2 | 4 | 6 |
| Ex. 16 | | 0,25 | 1 | 1 | 1 | 1 | 1 | 3 |
| Ex. 17 | S1 | 0,1 | 1 | 1 | 1 | 1 | 2 | 4 |
| Ex. 18 | | 0,25 | 1 | 1 | 1 | 1 | 1 | 3 |
| Ex. 19 | S2 | 0,1 | 1 | 1 | 1 | 2 | 2 | 5 |
| Ex. 20 | | 0,25 | 1 | 1 | 1 | 1 | 1 | 3 |

The inventive stabilizers, especially S1, show very good optical color impression during simulated heat stability test at 170°C and are much better than classical phenol-based stabilizers such as B215, or other HALS compounds. Further, S1 is more efficient than the classical B215, with 0.1 wt. % additvation of S1 the same optical stabilization performance can be achieved as with B215 at 0.25wt. % additivation.

### Example 10: Effect of S1 on process protection in high molecular weight PP-R

The PP-R and the additives (stabilizer and lubricant CaSt) in the amounts listed in Table 12 were premixed in a Kenwood mixer with K-shape mixing blade.

**Table 12: Formulation composition**

| | |
|---|---|
| A-1 | PP + 0.05% CaSt |
| A-2 | PP + 0.05% CaSt + 0.1% B 215 |
| A-3 | PP + 0.05% CaSt + 0.1% LS 770 |
| A-4 | PP + 0.05% CaSt + 0.25% LS 770 |
| A-5 | PP + 0.05% CaSt + 0.1% LS 944 |
| A-6 | PP + 0.05% CaSt + 0.25% LS 944 |
| A-7 | PP + 0.05% CaSt + 0.1% N 30 |
| A-8 | PP + 0.05% CaSt + 0.25% N 30 |
| A-9 | PP + 0.05% CaSt + 0.1% BB S-EED |
| A-10 | PP + 0.05% CaSt + 0.25% BB S-EED |

The premixed formulation was pre-extruded with a Collin single screw extruder (30 x 25D) at 210 °C with water bath and pelletized. The compounded pellets from this process are referred to as Pass 0.

The multiple extrusion test was carried out with all formulations to study the stabilizer impact on polymer protection during the multiple extrusion process. For multiple extrusion trials, the pellets from Pass 0 were extruded in a Lab Tech Single Screw Extruder (20 x 30D) at 270 °C in a setup with a water bath and pelletized for multiple passes. The samples were collected after 1 pass, 3 passes and 5 passes. The melt flow rate (MFR) and discoloration ΔE of the extrudates were analyzed. The results of the measurements are summarized in tables 13a and 13b below.

**Table 13a: MFR after multiple extrusion @270°c**

| **No.** | **Formulation** | **Stabilizer** | **Amount of Stabilizer** | **MFR (g/10 min)** | | | |
|---|---|---|---|---|---|---|---|
| | | | | Pass 0 | Pass 1 | Pass 3 | Pass 5 |
| Ex. 21 | A-1 | w/o | | 5,7 | 12,7 | 28,3 | 52,1 |
| Ex. 22 | A-2 | B 215 | 0.1% | 4,4 | 8,1 | 13,4 | 16,8 |
| Ex. 23 | A-3 | T770 | 0.1% | 2,8 | 4,6 | 5,9 | 7,8 |
| Ex. 24 | A-4 | | 0.25% | 1,5 | 1,9 | 2,6 | 3,4 |
| Ex. 25 | A-5 | C944 | 0.1% | 1,4 | 2,2 | 5,4 | 13,6 |
| Ex. 26 | A-6 | | 0.25% | 0,8 | 1,2 | 3,2 | 8,7 |
| Ex. 27 | A-7 | N 30 | 0.1% | 3,1 | 5,2 | 7,1 | 11,7 |
| Ex. 28 | A-8 | | 0.25% | 1,6 | 2,4 | 3,2 | 4,6 |
| Ex. 29 | A-9 | S1 | 0.1% | 2,1 | 3,1 | 3,9 | 5,7 |
| Ex. 30 | A-10 | | 0.25% | 1,1 | 1,4 | 2,1 | 3,0 |

The increasing melt flow rate is related to the chain degradation reaction due to the high processing temperatures. Surprisingly S1 has better process stabilization properties in multiple extrusion trials than the classical process stabilizing antioxidant B215 and other HALS stabilizers.

**Table 13b: Discoloration (ΔE) after multiple extrusion @270°c**

| **No.** | **Formulation** | **Stabilizer** | **Amount of Stabilizer** | **Discoloration (ΔE)** | | |
|---|---|---|---|---|---|---|
| | | | | Pass 1 | Pass 3 | Pass 5 |
| Ex. 21 | A-1 | w/o | | 2,5 | 4,4 | 4,6 |
| Ex. 22 | A-2 | B 215 | 0.1% | 3,0 | 4,6 | 6,1 |
| Ex. 23 | A-3 | T770 | 0.1% | 2,9 | 4,9 | 5,7 |
| Ex. 24 | A-4 | | 0.25% | 3,4 | 4,9 | 5,8 |
| Ex. 25 | A-5 | C944 | 0.1% | 4,3 | 5,1 | 6,1 |
| Ex. 26 | A-6 | | 0.25% | 5,8 | 7,3 | 7,6 |
| Ex. 27 | A-7 | N 30 | 0.1% | 3,0 | 4,3 | 5,3 |
| Ex. 28 | A-8 | | 0.25% | 3,2 | 5,7 | 7,2 |
| Ex. 29 | A-9 | S1 | 0.1% | 2,4 | 4,0 | 4,5 |
| Ex. 30 | A-10 | | 0.25% | 2,4 | 4,4 | 5,0 |

S1 shows the lowest discoloration and therefore best color stabilizing of all tested stabilizers during multiple extrusion processing.

## Claims

1. Compound of formula (II) 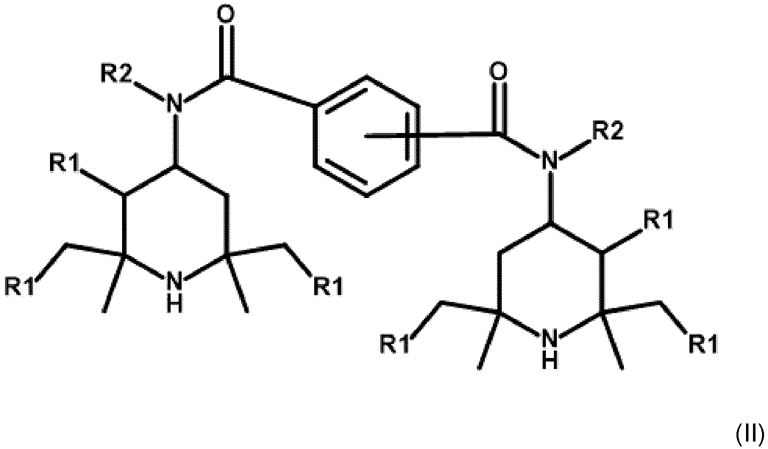 **characterized in, that**
R₁ is H, or a linear alkyl group having from 1 to 3 carbon atoms, preferably H, or a linear alkyl group having from 1 to 2 carbon atoms, and
R₂ is selected from linear, branched or cyclic alkyl groups having from 2 to 8 carbon atoms, preferably from 3 to 6 carbon atoms, particularly preferably from 4 to 5 carbon atoms.

2. Compound according to claim 1 wherein the compound of formula (II) is a compound of formula (IV): 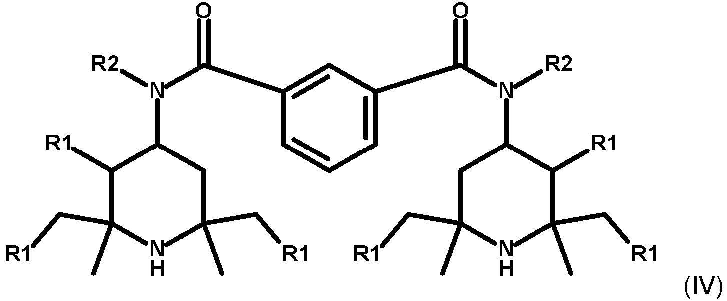 **characterized in, that**
R₁ is H, or a linear alkyl group having from 1 to 3 carbon atoms, preferably H, or a linear alkyl group having from 1 to 2 carbon atoms, and
R₂ is selected from linear, branched or cyclic alkyl groups having from 2 to 8 carbon atoms, preferably from 3 to 6 carbon atoms, particularly preferably from 4 to 5 carbon atoms.

3. Compound according to claim 1 **characterized in, that**
R₁ is hydrogen, and
R₂ is butyl, preferably n-butyl.

4. Compound according to claim 2 **characterized in, that**
R₁ is hydrogen, and
R₂ is butyl, preferably n-butyl.

5. Compound according to claim 2 **characterized in, that** R₂ is n-butyl.

6. Compound according to claim 5 in its α-phase, which is **characterized by** reflections with strong intensities in the x-ray powder diffractogram, measured with Cu-Kα₁ radiation in transmission mode on a STOE STADI P diffractometer, at 2θ angles of 11.3°, 11.9°, 13.7°, 14.2°, 16.2°, 18.6° and 20.8°, preferably with additional reflections with moderate intensities at 2θ angles of 6.8°, 7.2°, 9.0°, 10.3°, 13.3°, 15.1°, 16.7°, 17.9°, 20.1°, 21.9° and 24.9°.

7. Compound according to claim 5 in its β-phase, whereas the β-phase **characterized by** reflections with strong intensities in the x-ray powder diffractogram, measured with Cu-Kα₁ radiation in transmission mode on a STOE STADI P diffractometer, at 2θ angles of 11.6° and 15.0°, preferably with additional reflections with moderate intensities at 2θ angles of 18.9°, 19.6° and 20.7°, especially preferred with additional reflections with weak intensities at 2θ angles of 10.5°, 16.0°, 18.1°, 21.2°, 23.0° and 25.4°.

8. Compound according to claim 5 in its γ-phase, whereas the γ-phase is **characterized by** reflections with a very strong intensity in the x-ray powder diffractogram, measured with Cu-Kα₁ radiation in transmission mode on a STOE STADI P diffractometer, at a 2θ angle of 12.1°, and reflections with strong intensities at 2θ angles of 13.5°, 16.8° and 20.3°, and preferably with additional reflections with moderate intensities at 5.9°, 17.2° and 23.5°, and especially preferred with additional peaks of weak intensities at 2θ angles of 11.0°, 19.2° and 24.4°.

9. Compound according to claim 4 in its amorphous form, which is **characterized in** the DSC by a glass transition temperature Tg in the 1st heating curve between 26 - 32°C, measured according to DIN EN ISO 11357-2.

10. Process for the preparation of the compound according to any one of the preceding claims, comprising the following steps
a) provision of
ai) a non-methylated, sterically hindered amine of formula (III) 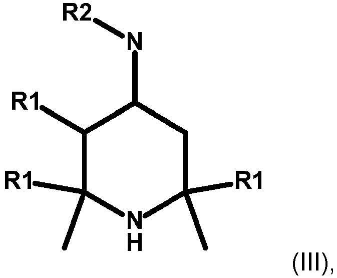 and
aii) a benzenedicarboxylic acid chloride or it's dimethyl ester (BCA), preferably isophthalic acid chloride or its dimethyl ester, particularly preferably isophthalic acid chloride (IPC)
b) condensation of
bi) the sterically hindered amine of formula (III), with
bii) benzenedicarboxylic acid chloride or its dimethyl ester, preferably isophthalic acid chloride or its dimethyl esters, particularly preferably isophthalic acid chloride (IPC).
**characterized in that** the substituents of the compound of formula (III) are as follows
R₁ is H or a linear alkyl group having from 1 to 3 carbon atoms,
preferably H or a linear alkyl group having from 1 to 2 carbon atoms; and
R₂ is selected from linear, branched or cyclic alkyl groups having from 2 to 8 carbon atoms, preferably from 3 to 6 carbon atoms,
particularly preferably from 4 to 5 carbon atoms.

11. Process according to claim 10 **characterized in, that**
R₁ is hydrogen, and
R₂ is butyl, preferably n-butyl.

12. Process according to claim 10 or 11, **characterized in that** the molar ration of BCA to formula (III) is from 1:1,8 to 1:2,5, preferably it is from 1:1,8 to 1:2,1, more preferably it is from 1:1,9 to 1:2.

13. Process according to one or more of claims 10 - 12, **characterized in that** the solvent is cyclohexane, xylene, toluene, chlorobenzene, ethanol, n-propanol or isopropanol or a mixture of 60 - 80 vol.% of isopropanol or xylene and 20 -40 vol.% of water.

14. Composition, comprising a compound according to any one of claims 1 to 9 and one or more thermoplastic or duroplastic polymers.

15. Composition according to claim 14, wherein the compound of formula (II) according to claim 1 to 9 is present in the composition in an amount of 0.01 to 50.0 wt%, preferably of 0.05 to 5.0 wt% or 10 to 40 wt%, more preferably of 0.08 to 2.5 wt% or 15 - 30 wt%, particulary preferably from 0.1 to 1.0 wt% or 20 - 25 wt%, based on the total weight (100 wt%) of the total composition.

16. Composition according to claim 14 to 15, wherein the thermoplastic or duroplastic polymer is selected from the group consisting of polyolefins, polyesters, polyester resins, polyurethanes, polyurethane resins, polyalkylene terephthalates, styrenic polymers, styrenic copolymers, acrylic polymers, acrylic resins, polyamides, epoxides, epoxide resins, polyester resins and alkyd resins.

17. Composition according to claim 16, wherein the thermoplastic polymer is one or more polyamides or one or more polyolefins, preferably PA 6 or PA 66 or one or more polyethylene or polypropylene homo- or copolymers.

18. Composition according to any one of claims 14 to 17, wherein the composition is a masterbatch or a coating system or a hot melt formulation.

19. Process for stabilizing a thermoplastic or duroplastic polymer, **characterized in that** a thermoplastic or duroplastic polymers is mixed with the compound according to any one of claims 1-9 in an amount of 0,01 wt% to 10 wt%, preferably of 0.05 to 5.0 wt%, more preferably of 0.08 to 2.5 wt%, particularly preferably in an amount of 0.1 to 1.0 wt%; and melt-processed.

20. Process for stabilizing a polymer, **characterized in that** the polymer is mixed with
a masterbatch, comprising the compound of formula (II) in an amount of 10 to 70 wt%, preferably of 20 to 60 wt%, more preferably of 20 to 40 wt%, based on the total weight (100 wt%) of the masterbatch,
in an amount of 0.015 wt% - 90 wt%, preferably from 0.02 wt% to 60 wt% more preferably from 0.05 wt% to 50 wt%, particularly preferably from 0.1 wt% to 40 wt%, based on the total weight of the total composition, and
melt-processed.

21. Process for stabilizing a polyamide, **characterized by** the following steps
(a) adding the compound of formula (II) to the one or more monomers used for the polymerization of the polyamide in an amount of 0,001 to 1 wt.%, preferably of 0.01 to 0.5 wt.%, more preferably of 0.05 to 0.3 wt.%; based on the total weight of the one or more monomers,
(b) polymerizing the one or more monomers to produce the polyamide.

22. Process according to claim 21, wherein the one or more monomers are selected from the group consisting of ε-caprolactam, ω-aminolauric acid, laurolactam, hexamethylenediamine, 11-aminoundecanoic acid, terephthalic acid, terephthalic chloride, isophthalic acid, isophthalic chloride, adipic acid, tetramethylenediamine, phenylenediamine, preferably selected from the group consisting of ε-caprolactam, hexamethylenediamine, adipic acid, more preferably selected from the group consisting of ε-caprolactam.

23. Use of the compound of formula (II) according to claim 1 to 9 to stabilize thermoplastic or duroplastic polymers against degradation, discoloration and/or yellowing of polymers caused by heat, light or oxidation.

24. Use according to claim 23, wherein the polymers are PA 6, PA 66, PP homopolymers, PP copolymer, PE homopolymers or PE copolymers.
